(19)

Europäisches Patentamt

European Patent Office

Office européen des brevets

(11) **EP 2 883 502 A2**

(12) **EUROPEAN PATENT APPLICATION**

(43) Date of publication:
**17.06.2015 Bulletin 2015/25**

(51) Int Cl.:
***A61B 8/08*** *(2006.01)*        ***A61B 8/13*** *(2006.01)*
***A61B 8/14*** *(2006.01)*        ***A61B 8/00*** *(2006.01)*

(21) Application number: **14198040.9**

(22) Date of filing: **15.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.12.2013 KR 20130156434**

(71) Applicant: **Samsung Electronics Co., Ltd**
**Gyeonggi-do 443-742 (KR)**

(72) Inventors:
• **Jung, Yun-sub**
**Gyeonggi-do (KR)**
• **Shim, Hwan**
**Gyeonggi-do (KR)**
• **Kim, Young-tae**
**Gyeonggi-do (KR)**
• **Lim, Hyung-joon**
**Seoul (KR)**
• **Cheon, Byeong-guen**
**Gyeonggi-do (KR)**

(74) Representative: **Appleyard Lees**
**15 Clare Road**
**Halifax HX1 2HY (GB)**

(54) **Method and Apparatus to Provide Blood Vessel Analysis Information Using Medical Image**

(57)    A blood vessel analysis information providing method includes emitting an ultrasonic signal on a body portion where a blood vessel exists and sensing a reflected ultrasonic signal, generating a color mode image by using the reflected ultrasonic signals, and determining diameters of the blood vessels based on pixel values of the generated color mode image.

# FIG. 1

100

EP 2 883 502 A2

**Description**

BACKGROUND OF THE INVENTION

1. Field of the Invention

Apparatuses and methods consistent with what is disclosed herein relate to methods of providing medical image analysis, and more specifically, to methods of analyzing blood vessel information in medical images obtained from at least one of various types of diagnosis equipment and providing the analysis result, and apparatuses using the method.

2. Description of the Related Art

Medical images obtained from medical image equipment, such as X-ray, CT, MRI, or ultrasonic wave equipment, are used for a diagnosis purpose in a variety of fields and are also frequently used in performing the medical treatment. A targeting subject and a corresponding method can be usable to perform the purpose of diagnosis or treatment. Bones and blood vessels are usually examined in medical images because they are distributed in most organs of the human body. Specifically, blood vessels are one of most landmarks in view of the anatomy since they are working in various patterns between human bodies or between organs while simultaneously being a center of functional roles for a living subject.

Such anatomic information of blood vessels is directly and indirectly used when medical images for the diagnosis and the treatment are obtained. For example, vector information of blood vessels is used to measure a blood flow rate by using Doppler effects (PW-Doppler). Further, information regarding blood vessel diameters, blood vessel vectors, and blood vessel positions are requested when the blood vessel intervention for treating the blood vessel related diseases is performed. Currently, the above-mentioned information is obtained depending on experienced knowledge or intuitive decision of the practitioners. However, the related information measuring methods may generate inter-observer or intra-observer errors, and cause the diagnosis to take more time, and give difficulties to building a systematic diagnosis.

Meanwhile, when measuring a blood flow rate by using ultrasonic wave equipment, it is necessary to delete an aliasing area generated by a frequency variation and setting a region of interest (ROI) in addition to measuring a blood vessel. The 'aliasing' indicates an area which is expressed differently from a surrounded blood flow area due to a blocking section of blood vessels when image display equipment displays blood vessels by using ultrasonic waves. The 'aliasing' is such a phenomenon in which bandwidths of Doppler spectrum are shifted because of a frequency variation and is expressed in different areas from surrounded blood flow areas on medical images.

One of the related technologies to delete aliasing is to shift a base line. This method is performed in a Doppler spectrum area and has a disadvantage in that aliasing cannot be directly deleted from a medical image. If it is possible to determine an aliasing area and restore the aliasing area to a blood vessel area, it may require complicated image processing and longer processing time.

Further, when setting an ROI, a user directly designates the ROI using the related technology. Passive setting by humans deteriorates reproducibility, and has a disadvantage of a longer diagnostic time. Whenever the ROI is set by a user, the result may vary according to a user preference or error.

There may be methods usable to measure blood vessel information. One method is to detect outer edges of blood vessels with an image processing and to estimate the blood vessels with the detected outer edges, and another method is to estimate vectors of blood vessels by fitting blood flow components expressed in C-mode images generated by ultrasonic waves. These related methods may have problems. First, regarding the method to detect outer edges, B-mode images of ultrasonic waves are generally used; B-mode images may have a lower SNR (signal versus noise or a signal-to-noise ratio), lower clarity in images, and vagueness in determining blood vessel components among the detected outer edge components. Further, the fitting method to use blood vessel components in C-mode images may have a problem in that vectors of blood vessels are estimated on a wrong direction when blood flow signals (color information) constituting C-mode images are insufficient. Lastly, all the related method to measure blood vessel information are developed for considering a case that there is one blood vessel in an ROI. Thus, when there are a plurality of blood vessels, the related methods have disadvantages in that information cannot be measured because they distinguish one blood vessel from the various blood vessels and selectively recognize the blood vessel.

Further, when treatment involves use of blood vessel intervention, angiography is applied so that a medical practitioner proceeds with the treatment, while directly viewing blood vessel angiographic images with his eyes. A medical practitioner moves a catheter to a lesion while viewing two-dimensional images in real time. Thus, a treatment time varies depending on experience and skill of a medical practitioner because of the above described process. Increasing the medical treatment time may lead to increasing radioactive exposure and additional injecting a contrast agent. A conventional method is just for providing positions of the catheter regarding the technology to provide information for the blood vessel intervention.

Further, when performing a bimodality image registration between different types of medical image equipment, indicators such as bifurcation and crossover of blood vessels are used as landmarks in view of blood vessels. Images having insufficiency for these indicators may have a problem in that blood vessels cannot be indicators for the registration.

SUMMARY OF THE INVENTION

The present general inventive concept provides a method of providing blood vessel analysis information to automatically set a region of interest (ROI) and an apparatus configured to provide blood vessel analysis information to display an image of a human body by using an ultrasonic wave.

The present general inventive concept also provides a method of providing blood vessel analysis information that can detect a diameter and an of a blood vessel regarding an ROI precisely even when there are one or more blood vessels, and an apparatus configured to provide blood vessel analysis information thereof.

The present general inventive concept also provides a method of providing blood vessel analysis information that can detect and interpolate an aliasing area more precisely in a blood vessel image and an apparatus thereof.

Additional features and utilities of the present general inventive concept will be set forth in part in the description which follows and, in part, will be obvious from the description, or may be learned by practice of the general inventive concept.

The foregoing and/or other features and utilities of the present general inventive concept may be achieved by providing a method of providing blood vessel analysis information, the method including emitting an ultrasonic signal on a body portion corresponding to a blood vessel and sensing a reflected ultrasonic signal, generating a color mode image by using the sensed ultrasonic signal, and determining a diameter of the blood vessel based on one or more pixel values of the generated color mode image. The determining the diameter of the blood vessel may include determining a shortest distance of a blood flow area as the diameter of the blood vessel, by comparing one or more profile values regarding the pixel values on one or more virtual lines crossing over the blood vessel in the generated color mode image.

The method may further include emitting and sensing an ultrasonic wave, generating a color mode image, and determining an angle of a blood vessel based on one or more pixel values of the generated color mode image.

The method may further include determining an angle of the blood vessel based on the one or more pixel values on one or more virtual lines crossing over the determined diameter.

The determining angles of the blood vessels may include adding one or more pixel values on a plurality of virtual lines crossing over the determined diameter according to a corresponding sliding angle from a horizontal plane, and determining an angle of the blood vessel based on the sliding angles regarding the plurality of virtual lines and the smallest value of the added pixel values.

The method may further include emitting and sensing an ultrasonic signal, generating a color mode image, and establishing an ROI regarding one or more portions of the generated color mode image. Further, the method may further include detecting an aliasing area whose one or more pixel values are out of a preset pixel value in a blood flow area of the ROI, determining a diameter of the blood vessel based on one or more pixel values of an ROI image, and determining an angle of the blood vessel based on one or more pixel values on one or more virtual lines crossing over the determined diameter.

The establishing the ROI may include establishing the ROI on a previously established position of the generated color mode image, and adjusting the ROI so that a center coordinate thereof is approached to a center coordinate of a blood flow, when a center coordinate of the ROI is out of a preset range from the center coordinate of the blood flow in the generated color mode image.

Further, the method may be performed on the ROI established regarding one or more portions of the generated color mode image, and the ROI may be newly established as a next or new ROI based on the determined angle of the blood vessel when image processing regarding the ROI completes.

Further, the detecting the aliasing area may include detecting the aliasing area by further considering a histogram of the generated color mode image.

Further, the detecting the aliasing area may include performing a labeling operation in each section of the generated color mode image according to one or more pixel values of the generated color mode image, performing a clustering operation regarding the labeled section based on one or more pixel values of the generated color mode image, determining the aliasing area based on a result of the clustering operation, and interpolating the determined aliasing area.

The determining the aliasing area may include determining the labeled section as the aliasing area when there are more than two clustering areas within one labeled section.

The foregoing and/or other features and utilities of the present general inventive concept may be achieved by providing a computer-readable medium containing computer-readable codes as a program to perform a method described above or hereinafter.

The foregoing and/or other features and utilities of the present general inventive concept may be achieved by providing an apparatus to provide blood vessel analysis information, the apparatus including a display, an ultrasonic transmitter and receiver configured to emit an ultrasonic signal on an object and sense a reflecting ultrasonic signal, and a controller configured to generate a color mode image by using the reflecting ultrasonic signal and determine a diameter of a blood vessel based on one or more pixel values of the generated color mode image.

The controller may determine a shortest distance of a blood flow area as a diameter of the blood vessel by comparing one or more profile values regarding one or more pixel values on one or more virtual lines crossing over the blood vessel in the generated color mode image.

The controller may determine an angle of the blood vessel based on one or more pixel values of one or more virtual lines crossing over the determined diameter.

The controller may add one or more pixel values on a plurality of virtual lines crossing over the determined diameter according to a corresponding sliding angle from a horizontal plane, and determine the angle of the blood vessel based on sliding angles regarding a plurality of virtual lines and the smallest value of the added pixel values.

The controller may establish an ROI having an image regarding a center of the blood vessel in one or more portions of the generated color mode image, and perform image processing regarding the ROI.

The controller may establish the ROI on a previously established position of the generated color mode image, and adjust the ROI so that a center coordinate thereof is approached to a center coordinate of a blood flow when a center coordinate of the ROI is out of a preset range from the center coordinate of the blood flow in the generated color mode image.

The controller may operate regarding the ROI established on one or more portions of the generated color mode image, and a newly establish ROI based on the determined angle of the blood vessel when image processing regarding the ROI completes.

The controller may distinguish a blood flow area based on one or more pixel values of the generated color mode image and detect an aliasing area whose pixel values are out of a preset pixel value in the distinguished blood flow area.

The controller may detect the aliasing area by further considering a histogram of the generated color mode image.

The controller may perform a labeling operation on each section of the generated color mode image according to one or more pixel values of the generated color mode image, perform a clustering operation regarding the labeled sections based on one or more pixel values of the generated color mode image, determine an aliasing area based on a result of the clustering operation, and interpolate the determined aliasing area.

Further, when there are more than two clustering areas within one labeled section, the controller may determine the labeled section to be an aliasing area.

The foregoing and/or other features and utilities of the present general inventive concept may be achieved by providing a method or apparatus to provide blood vessel analysis information to automatically establish an ROI to display a body image with a ultrasonic wave.

The foregoing and/or other features and utilities of the present general inventive concept may be achieved by providing a method or apparatus to provide blood vessel analysis information to detect a diameter and/or an angle of a blood vessel in an ROI precisely even when there are one or more blood vessels.

The foregoing and/or other features and utilities of the present general inventive concept may be achieved by providing a method or apparatus to provide blood vessel analysis information to detect and interpolate an aliasing area precisely in a blood vessel image.

The foregoing and/or other features and utilities of the present general inventive concept may be achieved by providing a method or apparatus to provide a non-transitory computer-readable medium to contain computer-readable codes as a program to execute a method described above or hereinafter.

The foregoing and/or other features and utilities of the present general inventive concept may be achieved by providing a method or apparatus to provide a method of providing blood vessel analysis information, the method including receiving a signal reflected from a body portion, displaying a color mode image on a screen of a display according to the received signal, and automatically outputting a diameter of the blood vessel according to a determination based on one or more pixel values of the generated color mode image.

The outputting the diameter of the blood vessel may include displaying the diameter over the color mode image on the screen of the display.

The method may further include automatically displaying a region of interest on the screen of the display according to information on a blood flow of the blood vessel.

The method may further include generating a new region of interest along the blood vessel according to a portion of the image corresponding to an external catheter.

The method may further include automatically displaying one or more region of interests each with at least one of a diameter and an angle of the blood vessel to correspond to the displayed region of interest.

The method may further include automatically displaying at least one angle of the blood vessel according to the pixel values of virtual lines passing an area corresponding to the diameter.

The method may further include automatically displaying an aliasing area with interpolated data using adjacent area of the blood vessel.

The foregoing and/or other features and utilities of the present general inventive concept may be achieved by providing a method or apparatus to provide an apparatus to provide blood vessel analysis information, the apparatus including a receiver configured to receive a signal reflected from a body portion, and a controller configured to generate a color mode image according to the received signal, to display the color mode image on a screen of a display, to determine a diameter of a blood vessel based on one or more pixel values of the generated color mode image, and to display the determined diameter on the screen of the display.

The controller may control the display to display the diameter of the blood vessel on the image included in the screen of display.

The controller may control the display to automatically display a region of interest on the screen of the display according to information on a blood flow of the blood vessel.

The controller may automatically generate and display a new region of interest along the blood vessel according to a portion of the image corresponding to an external catheter.

The controller may sequentially display one or more region of interests each with a diameter and an angle of the blood vessel to correspond to the displayed region of interest.

The controller may automatically generate and display at least one angle of the blood vessel according to the pixel values of virtual lines passing an area corresponding to the diameter.

The controller may automatically determine and display an aliasing area with interpolated data using adjacent area of the blood vessel.

BRIEF DESCRIPTION OF THE DRAWINGS

The above and/or other features and utilities of the present general inventive concept will be more apparent by describing certain exemplary embodiments of the present inventive concept with reference to the accompanying drawings, in which:

FIG. 1 is a block diagram of a blood vessel analysis information providing apparatus according to an embodiment of the present general inventive concept;

FIG. 2 illustrates a method of generating a C-mode image;

FIG. 3 illustrates a method of measuring a diameter and an angle of a blood vessel according to an embodiment of the present general inventive concept;

FIG. 4 illustrates a plurality of virtual lines which pass over a diameter of a blood vessel;

FIG. 5 is a diagram to illustrate that an ROI (region of interest) is established according to an embodiment of the present general inventive concept;

FIG. 6 illustrates a method of consecutively setting an ROI by considering an angle of a blood vessel;

FIGS. 7 and 8 illustrate detecting and interpolating an aliasing area according to an embodiment of the present general inventive concept;

FIGS. 9 to 11 are flowcharts illustrating a method of providing blood vessel analysis information according to an embodiment of the present general inventive concept;

FIG. 12 is a diagram illustrating an ultrasonic diagnostic apparatus according to an embodiment of the present general inventive concept;

FIG. 13 illustrates an ultrasonic diagnostic process with the ultrasonic diagnostic apparatus of FIG. 12;

FIG. 14 is a diagram illustrating detection of a diameter and angle of a blood vessel by using the ultrasonic diagnostic apparatus of FIG. 12;

FIG. 15 illustrates a moving path from a top-casting position of a catheter;

FIG. 16 is a block diagram illustrating a blood vessel angiographic image apparatus according to an embodiment of the present general inventive concept;

FIG. 17 illustrates a screen of a catheter moving path according to an embodiment of the present general inventive concept;

FIG. 18 is a flowchart illustrating a blood vessel angiographic image display method according to an embodiment of the present general inventive concept;

FIG. 19 illustrates views photographed by different apparatuses regarding a same portion of a human body;

FIG. 20 is a block diagram illustrating a medical image registering apparatus according to an embodiment of the present general inventive concept;

FIG. 21 illustrates angles of blood vessel bifurcations and distances between blood vessel bifurcations;

FIG. 22 illustrates detecting one or more landmarks for image registration; and

FIG. 23 is a flowchart illustrating a method of designating one or more image registration landmarks according to an embodiment of the present general inventive concept.

## DETAILED DESCRIPTION OF THE PREFERRED EMBODIMENTS

Reference will now be made in detail to the embodiments of the present general inventive concept, examples of which are illustrated in the accompanying drawings, wherein like reference numerals refer to the like elements throughout. The embodiments are described below in order to explain the present general inventive concept while referring to the figures.

The matters defined in the description, such as detailed construction and elements, are provided to assist in a comprehensive understanding of the present inventive concept. Accordingly, it is apparent that the exemplary embodiments of the present inventive concept can be carried out without those specifically defined matters. Also, well-known functions or constructions are not described in detail since they would obscure the invention with unnecessary detail.

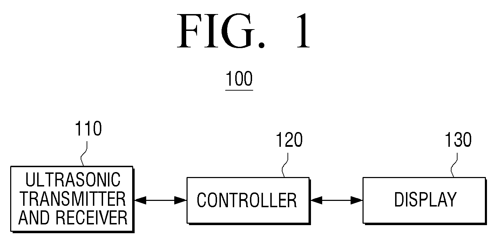

FIG. 1 is a block diagram illustrating a blood vessel analysis information providing apparatus 100 according to an embodiment of the present general inventive concept.

Referring to FIG. 1, the blood vessel analysis information providing apparatus 100 according to an embodiment includes an ultrasonic transmitter and receiver 110, a controller 120, and a display 130.

The ultrasonic transmitter and receiver 110 is a unit to emit an ultrasonic signal on a portion of a human body corresponding to a flow of a blood vessel flow and to sense an ultrasonic echo signal reflecting from the portion of a human body. For the above, the ultrasonic transmitter and receiver 110 may include a probe constituted with a transducer including a vibrator and a tuning coil to sense the ultrasonic echo signal. Further, the ultrasonic transmitter and receiver 110 may include an analogue/digital converter and a signal processor to process the received signal.

The display 130 is provided to display one or more images processed by the controller 120. The display 130 may include various display panels. Thus, the display 130 may be implemented to be various display technologies such as organic light emitting diodes (OLED), liquid crystal display panel (LCD panel), plasma display panel (PDP), vacuum fluorescent display (VFD), field emission display (FED), and electro luminescence display (ELD). The display panel may be an emitting type; however, a reflecting type (E-ink, P-ink, photonic crystal) may be used as the display panel. Further, the display panel may be a flexible display or a transparent display, and it may include a plurality of display panels. The present general inventive concept is not limited thereto. It is possible that a touch panel can be usable as the display 130 to display an image through a screen thereof and to receive a user input from a user through the screen to control a function of the apparatus 100.

The controller 120 controls overall operations of the blood vessel analysis information providing apparatus 100.

The controller 120 may include hardware constituted of a micro processing unit (MPU) or a central processing unit (CPU), a cache memory, and a data bus, and software (program) constituted of an operating system and one or more applications to perform corresponding purposes. One or more controlling commands regarding each unit for the operation of the blood vessel analysis information providing apparatus 100 are read in a memory according to a system clock, an electrical signal is generated according to the read controlling commands to operate a corresponding unit of the hardware.

The controller 120 controls an ultrasonic trans-receiving operation of the ultrasonic transmitter and receiver 110. Further, when an ultrasonic signal reflected from one or more portions of a human body is received, the controller 120 generates a B-mode image having black and white signals or a C-mode (color mode) image by using the received signal.

The B-mode (Brightness-mode) image is an image corresponding to a portion of a human body where the ultrasonic waves are emitted in black and white by using an ultrasonic echo signal reflecting from the portion of the human body. When a distance from the portion of a human body is represented on a horizontal axis and a width of the reflected echo signal is represented on a vertical axis, the width may be substituted and expressed with brightness of dots. The B-mode image may be constituted of a black and white image.

The C-mode (color Doppler mode) image is an image corresponding to a portion of a human body where the ultrasonic wave is emitted in one or more colors by using the ultrasonic echo signal reflected from the portion of a human body. The controller 120 may measure a blood flow rate by calculating one or more variant frequencies when the ultrasonic echo signal is received and the frequency variations occur due to Doppler effects. Further, the C-mode image may be constituted by using the above described components.

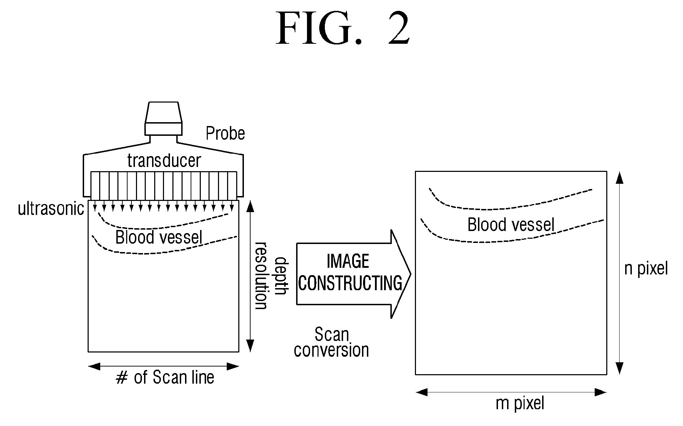

FIG. 2 illustrates a method of generating a C-mode image in an apparatus, for example, the blood vessel analysis information providing apparatus 100 of FIG. 1.

The controller 120 selects one or more pixels, for example, m x n pixels surrounded a range gate of a Doppler beam. The m x n pixels may be selected by a user input or by covering a range including all of upper and the lower edges after automatically searching the upper and lower edges of a blood vessel wall. At this process, the blood vessel wall may be detected by using edge detection algorithm or homogeneity detection algorithm. Further, an ROI (region of interest) designating method which will be described below may be used.

Referring to FIG. 2, a velocity of an ultrasonic response signal regarding each pixel may have a pixel value between 0 and k (k is integer). k is determined by a bit resolution; for example, if the bit resolution is 8 bits of a resolution, each pixel may be expressed in a value (or color value) of 0 ~ 255.

The controller 120 may measure a diameter and/or an angle of a blood vessel based on one or more pixel values of the generated color mode image.

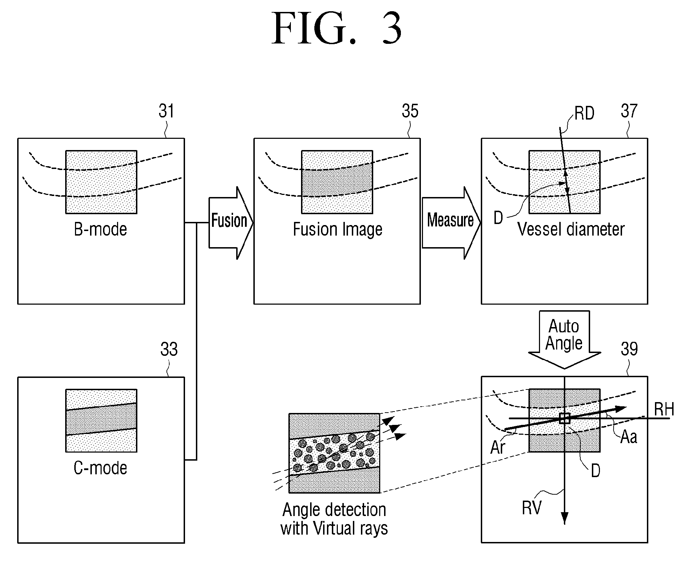

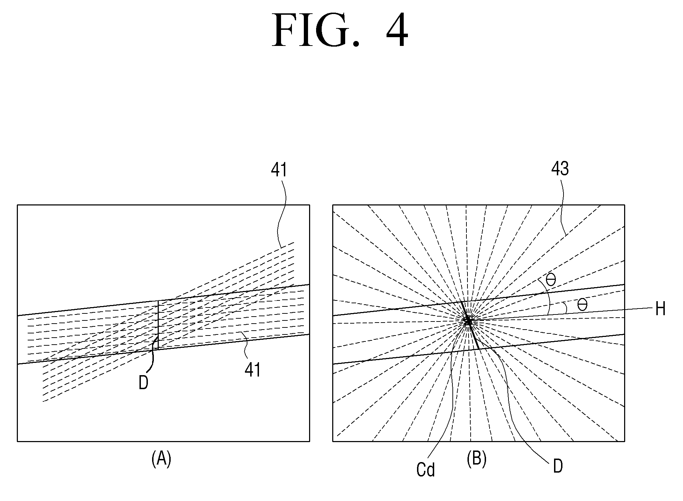

FIG. 3 illustrates a method of measuring a diameter and an angle of a blood vessel, and FIG. 4 illustrates a plurality of virtual lines crossing over a line or area corresponding to the diameter of the blood vessel.

Referring to FIGS. 1 through 4, the controller 120 determines a diameter of a blood vessel based on one or more pixel values of the color mode image. Further, the controller 120 may use the C-mode image and the B-mode image together to measure the diameter and/or the angle. At this process, reconstituting the generated images is performed. The embodiment of FIG. 3 illustrates such implementation.

Thus, a new image is generated by combining the B-mode image and the C-mode image. This method may compensate for a disadvantage that the B-mode image and/or the C-mode image may have. It may be difficult to find a visible boundary due to noise even though an anatomical boundary of a blood vessel and a surrounded organ cell can be viewed in the B-mode image. Further, color information may be insufficient even though a relative velocity of a blood flow can be viewed in the C-mode image. The blood vessel information is estimated by minimizing noise that can occur in the B-mode image and/or the C-mode image by using the reconstituted image.

Newly reconstituted image information may be calculated according to a following mathematical formula.

$$I_{f\text{-}image}(i,j) = \alpha * N_{b\text{-}image}(i,j) + \beta * N_{c\text{-}image}(i,j)$$

where $I_{f\text{-}image}$ is a reconstituted image (fusion image),

$N_{b\text{-}image}$, $N_{c\text{-}image}$ are normalized B-mode and C-mode images,

$\alpha$, $\beta$ are a weight factor, and

(i, j) is a pixel position when an image size is m*n ($0 \leq i \leq m, 0 \leq j \leq n$)

For more efficient calculation, image reconstituting may be performed only on an ROI which will be described below.

The controller 120 may determine a diameter (distance) of the blood vessel having a shortest blood flow area profile value as the diameter of the above blood vessel by comparing profile values of blood flow areas regarding one or more virtual lines crossing over one or more random pixels of the blood vessel. Herein, the profile values of the blood flow areas indicate adding one or more values of consecutive pixel values corresponding to the blood flow areas.

In the C-mode image or the reconstituted image, pixel values on a plurality of virtual lines crossing over the blood vessel may distinguish the blood flow areas and other areas based on a preset threshold. The blood flow areas are expressed in different values compared to other areas because of a blood flow rate. Therefore, the profile values may be constituted of the pixel values. At this process, a shortest line disposed on an area whose profile value corresponds to at least one of the blood flow areas may be determined as a traverse line RD disposed orthogonal to the blood vessel. Further, a length of the profile value area having the shortest line of the traverse line RD becomes a diameter D of the blood vessel as illustrated in FIG. 3.

When an ROI is set, a diameter of the blood vessel (Δv) is measured in a range gate which is a center coordinate of the blood flow in the ROI.

Next, the controller 120 determines an angle Aa of the blood vessel in a direction Ar with respect to a reference line, for example, a horizontal line RH perpendicular to a vertical line RV, as illustrated in FIG. 3. The controller 120 determines the angle Aa of the blood vessel based on one or more pixel values on one or more virtual lines crossing over the determined diameter D. As illustrated in a view A of FIG. 4, virtual lines 41 crossing over the determined diameter may be considered to determine an angle of the blood vessel. If there are virtual lines 41 passing through a center area of the blood vessel, the virtual lines 41 may be vectors of the blood vessel, and one of angles between the virtual lines and the horizontal plane may be an angle of the blood vessel. The virtual lines may be distinguished from each other.

At this process, as illustrated in a view A of FIG. 4, it may consider a plurality of virtual lines 41 crossing over the determined diameter D. If the number of virtual lines is $N_{VR}$, $N_{VR}$ and the determined diameter D, for example, Δv, satisfy a following relation.

$$N_{VR} \propto \Delta v / \lambda$$

where λ is a width between a plurality of virtual lines.

Thus, the number of virtual lines is inverse proportional to the width (or gap) of the adjacent virtual lines.

A plurality of virtual lines having various angles crossing over the diameter may be considered to determine an angle of the blood vessel. A view B of FIG. 4 illustrates virtual lines 43. Herein, a width of each virtual line may be generated by having a width of k° in a range from -180° to 180°. As a k value is smaller, a resolution of the calculated angle may be greater; however, a small k value may deteriorate a calculating speed. Herein, k may be used to be 1° and can be managed automatically or manually according to performance of an angle detecting operation or a user design or preference.

At this process, the controller 120 may add one or more pixel values regarding a plurality of virtual lines crossing over the determined diameter according to a sliding angle from a horizontal plane. In a view A of FIG. 4, it may add one or more pixel values of the virtual lines 41 having the same angle and crossing over the diameter D. Further, the controller 120 may determine an angle of the blood vessel based on the sliding angles regarding a plurality of virtual lines and the smallest value among the added values of the pixel values. For example, if the adding result of the pixel values on the virtual lines crossing over the diameter D and having a small angle from the horizontal plane is less than the added result of the pixel values on the virtual lines crossing over the diameter and having different angles in a view A of FIG. 4, an angle corresponding to the former can be an angle of the blood vessel.

A representative value can be defined by mathematically formulizing the above described values or components. Thus, the controller 120 may calculate a representative value regarding a plurality of virtual lines 43 crossing over a center Cd of the determined diameter D according to corresponding sliding angles θ from a horizontal plane H. A representative value is defined by a following mathematical formula.

$$E(\theta) = \Sigma^{NVR}_{i=1}(L_{i\_AVE} + k^* L_{i\_SD}) \ (-180 \leq \theta \leq 180)$$

where E(θ) is a representative value regarding a plurality of virtual lines having a sliding angle (θ) from the horizontal plane,

$N_{VR}$ is the number of virtual lines having a sliding angle (θ) from the horizontal plane,

$L_{i\_AVE}$ is an average value of pixel values on a virtual line at an i position having a sliding angle (θ) from the horizontal plane, and

$L_{i\_SD}$ is a standard deviation of pixel values on a virtual line at an i position having a sliding angle (θ) from the horizontal plane.

Further, when the sliding angle among angles of the plurality of virtual lines with respect to the horizontal plane has a smallest calculated representative value, the controller 120 determines the sliding angle of the virtual line as an angle of the blood vessel. The horizontal plane is disposed between boundary walls of the blood vessel displayed on a screen of a display.

The following will explain a method of setting an ROI.

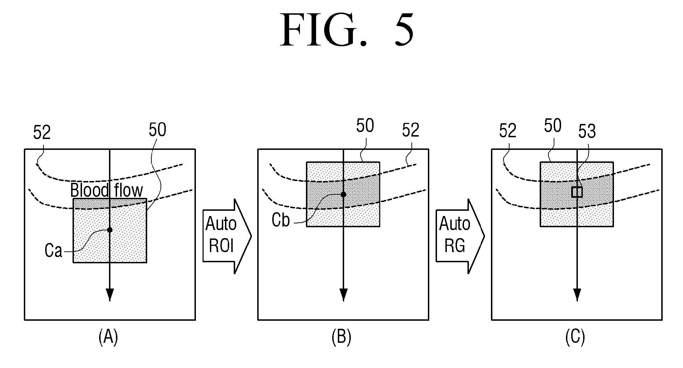

FIG. 5 illustrates the setting the ROI according to an embodiment of the present general inventive concept.

Referring to FIGS. 1 through 5, the controller 120 may set an ROI regarding one or more portions of the generated color mode image. The ROI indicates a portion of a human body where the ultrasonic signal is projected, and may include an image regarding a center of a blood vessel. However, the present general inventive concept is not limited thereto. It is possible that the ROI can be a portion around the center of the blood vessel. The method of providing blood vessel analysis information according to an embodiment may be performed on an established ROI.

The controller 120 establishes an ROI regarding a previously established position of the generated color mode image. Further, the controller 120 determines whether a center coordinate value of an ROI is out of a center coordinate value of a blood flow in the generated color mode image. If the center coordinate value of the ROI is within a previously established range from or similar to the center coordinate value of the blood flow, image processing may be performed. However, if the center coordinate value of the ROI is out of the previously established position from the center coordinate value of the blood flow in the generated color mode image, the ROI is adjusted so that the center coordinate value of the ROI is approached to the center coordinate value of the blood flow.

In a view A of FIG. 5, an initial ROI 50 includes only a portion of the blood vessel image 52. In this case, because a center coordinate value Ca regarding the initial ROI 50 is not within a range from or similar to a center coordinate value Cb of a blood flow of the blood vessel 52, the initial ROI 50 is adjusted as illustrated in a view B of FIG. 5. As a result of adjusting the ROI 50, the center coordinate value Ca of the initial ROI 50 may become within a range from or similar to the center coordinate value Cb of the blood flow. The center coordinate value Ca and the center coordinate value Cb may correspond to a center coordinate value 53 as illustrated in a view C of FIG. 5.

ROIs may be different according to clinical determining standards and corresponding diseases. Further, ROIs may be different according to a health condition, age, food digesting status, body condition, or body portion of a patient.

A location or a dimension (area) of an ROI may be set automatically or manually according to a user preference or design or may be adjusted according to a corresponding usage or a characteristic of the ROI and/or the image. The characteristic may be a resolution, color or outline.

The controller 120 may control the display 130 to display the views A, B, and/or C of FIG. 5 on a screen thereof. It is possible that the views A, B, and C of FIG. 5 can be sequentially displayed or can be simultaneously displayed on the screen thereof. It is also possible that only one view B or C can be displayed on the display 130 when an adjusting process to adjust the location of the ROI is performed in the controller 120.

A center position of the ROI may correspond to a center of an image screen (or a displayed image) as illustrated in the view A of FIG. 5. However, when the center position of the ROI is adjusted close to a blood vessel portion, for example, a center position of the blood flow or a center position of the blood vessel, the center position of the adjusted ROI may not be in a center area of the image screen. In this case, the controller 120 performs another adjusting process such that the display 130 can display the image with a center portion thereof to correspond to the center position of the adjusted ROI. That is, the center coordinate value 53 of the view C of FIG. 5 may correspond to a center area of the screen (or a displayed image) thereof.

It is also possible that the controller 120 may perform an adjusting process to move the center position of the blood flow to correspond to the center position of the ROI when the ROI is disposed in a center area of the image, rather than adjusting the position of the ROI. In this case, the controller 120 performs an adjusting process to adjust the image so that the center position of the blood flow can correspond to the center position of the ROI without moving the position of the ROI. Also the controller 120 may not have to perform the another adjusting process to change the centers of the ROI and the blood flow to correspond to the center area of the image.

When image processing regarding the established ROI completes, a next ROI may be set and image processing may be performed.

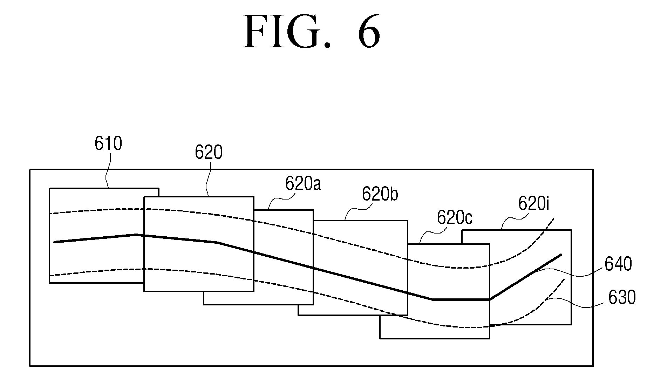

FIG. 6 illustrates a method of consecutively setting one or more ROIs according to corresponding angles of a blood vessel.

In order to move the initial ROI to a next ROI (or generate a next ROI) for image-processing after image processing regarding the established ROI, the controller 120 may perform a process to establish the new ROI based on the determined angle of a blood vessel described above.

FIG. 6 illustrates a process of completing image processing of an initial ROI 610 and setting a next ROI 620, 620a, 620b, 620c...and/or 620i. An ROI may be established by considering an angle of a blood vessel at a corresponding location of the ROI.

The controller 120 may set absolute or relative locations of the ROIs according to a user preference or corresponding usage. The dimensions (areas) of the ROIs may be the same or may be differently set according to a user preference or corresponding usage. The initial ROI and one or more ROIs may be sequentially displayed on an image screen of the display 130 according to the respective process to set a new ROI. All of the ROIs may be simultaneously displayed when a last ROI is set. The ROIs may overlap depending on the positions and dimensions thereof. The ROIs may be superimposed. Only a currently set (established or generated) ROI may be displayed with highlight or emphasis on the displayed image. The ROI may have different brightness or different boundary lines (outlines) with different thicknesses from other ROIs although FIG. 6 illustrates a same boundary thickness. It is possible that only one of the ROI, for example, a currently set ROI (initial ROI or new ROI) is displayed with a solid line, and previous ROi may be displayed with a broken line or disappeared from the displayed image. In this FIG. 6 also illustrates a blood vessel 630 and a center line 640 of a blood flow in the blood vessel 630. The blood flow center line 640 may correspond to a change of angles of the blood flow.

The following will explain an embodiment of detecting an aliasing area and interpolating an image thereof.

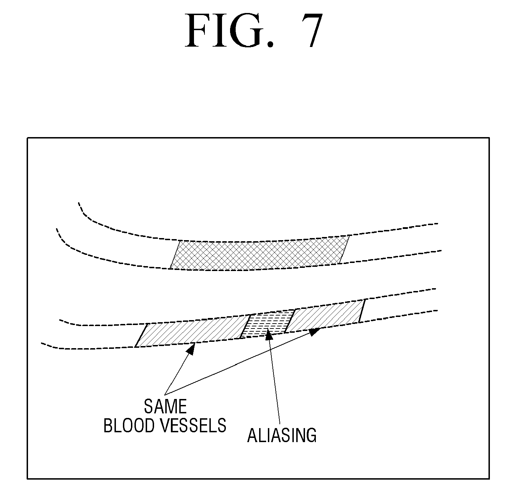

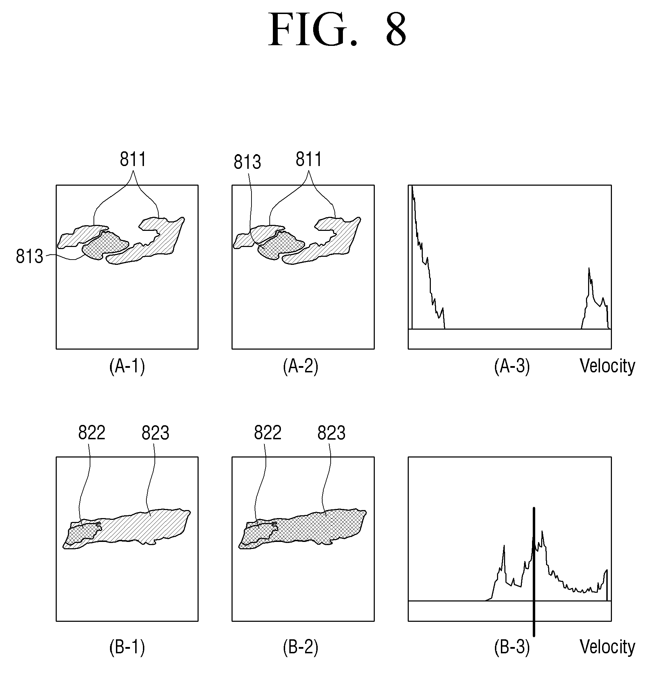

FIGS. 7 and 8 illustrate a method of detecting and interpolating an aliasing area.

The controller 120 may distinguish blood flow areas based on pixel values of a generated color mode image. Further, a reconstituted image can be usable to distinguish the blood flow areas as described above. The controller 120 detects one or more aliasing areas that are out of a preset pixel value on one or more blood flow areas. Pixel values of the blood flow areas may be differently established according to a blood flow rate; however, a pixel value may be higher than a critical value of a blood flow rate on a blood flow area. That is, a blocking section may be observed or detected because there may be an exterior material in a blood vessel, or noise may occur due to other reasons. Such a blocking section may be referred to as an aliasing area to be detected and interpolated. The aliasing area has a phenomenon in which a pixel value is shifted to a certain value higher than the critical value due to a bias, not by a value of a blood flow rate.

Referring to FIG. 7, an aliasing area has different pixel values compared to other blood flow areas. It is necessary to interpolate pixel values of other blood flow areas to create (generate) pixel values of the aliasing area.

However, since there may be various reasons why aliasing areas appear, it is necessary to determine whether the interpolating is requested because simple noise may cause aliasing.

A view A-1 of FIG. 8 illustrates an area 813 having different pixel values from a blood flow area 811. However, when a histogram of the generated color mode image illustrated in a view A-3 of FIG. 8 is considered, the above two areas have different blood flow velocities from each other. Thus, the areas 811 and 813 can be determined to be different blood vessels from each other. A view A-1 of FIG. 8 is an image in which the two blood vessels overlap and pass. Therefore, the two blood flow areas 811 and 813 are differently expressed also in the image-processed image as illustrated in a view A-2 of FIG. 8.

Referring to a view B-1 of FIG. 8, since two areas 822 and 823 are a same blood vessel according to a histogram illustrated in a view B-3 of FIG. 8, the two areas 822 and 823 are determined as an aliasing area and then interpolating is performed. Thus, the two blood flow areas 822 and 823 are uniformly expressed also in the image-processed image as illustrated in a view B-2 of FIG. 8.

When an aliasing area is detected, a following algorithm can be considered. First, the controller 120 performs a labeling operation on each section regarding the generated color mode images according to pixel values of the generated color mode image. The labeling operation is performed to assign a same label to consecutive pixels and different labels to other components (pixels). Further, the controller 120 performs a clustering operation on the labeled sections based on the pixel values of the generated color mode image. Next, an aliasing area is determined based on a result of the clustering operation. At this process, where there are more than two clustering areas within one labeled section, the labeled section may be determined as an aliasing area. The controller 120 interpolates the determined aliasing area using the pixel values of the adjacent area.

Meanwhile, a blood vessel may be connected in a consecutive vessel shape, not an independent spot shape. Thus, a small spot size of a label is considered to be noise and removed. The labels remaining after removing a noise component are all the components constituting one or more blood vessels. Velocity distribution is calculated by performing the clustering operation based on color information (blood flow rate) in each label.

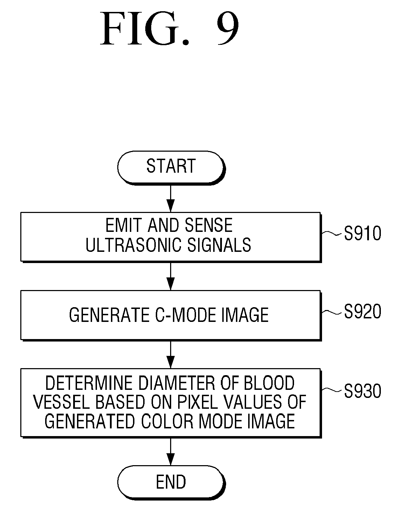

FIGS. 9 to 11 are flowcharts illustrating a method of providing blood vessel analysis information according to an embodiment of the present general inventive concept.

Referring to FIG. 9, the method of providing the blood vessel analysis information according to an embodiment may include emitting a first ultrasonic signal on a body portion corresponding to a blood vessel and sensing a second ultrasonic signal reflected from the body portion at operation S910, generating a color mode (C-mode) image by using the second ultrasonic signal at operation S920, and determining a diameter of the blood vessel based on one or more pixel values of the generated color mode image at operation S930. The operation S930 of determining the diameter of the blood vessel may include comparing one or more profile values regarding one or more pixel values of one or more virtual lines crossing over the blood vessel in the generated color mode image and determining a shortest distance (width) of a blood flow area among distances (widths) of the blood flow areas as a diameter of the blood vessel. The diameter of the blood vessel may be a single diameter of the vessel at a position thereof. However, the blood vessel may have a plurality of diameters at corresponding positions thereof along a blood flow of the blood vessel. The one or more diameters may be displayed at corresponding positions of the blood vessel on a screen of the display 130 according to a control signal of the controller 120.

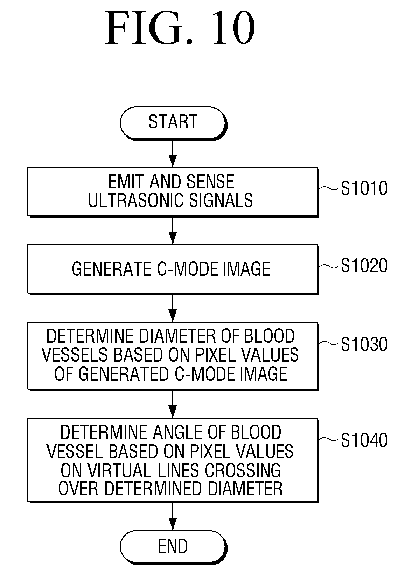

Referring to FIG. 10, the method of providing the blood vessel analysis information may include emitting and sensing ultrasonic signals at operation S1010, generating a color mode image using the sensed ultrasonic signal at operation S1020, and determining a diameter of a blood vessel based on one or more pixel values of the generated color mode image at operation S1030. The operations S1010, S1020, and S1030 may respectively correspond to the operations S910, S920, and S930.

The method may further include determining one or more angles of the blood vessel based on one or more pixel values on virtual lines crossing over the corresponding diameters at operation S1040.

Herein, the operation S1040 of determining the angle of the blood vessel may include adding pixel values on a plurality of virtual lines crossing over the determined diameter according to a sliding angle from a horizontal plane and determining the angle of the blood vessel based on one or more sliding angles regarding a plurality of virtual lines and the smallest value of the added pixel values. The one or more angles of the blood vessel may be displayed on a screen thereof according to a control signal of the controller 120. Since one or more angles can be obtained according to a shape of the blood vessel, the angles may be displayed at corresponding positions of the blood vessel on a screen of the display 130 when the controller 120 determines the corresponding angles.

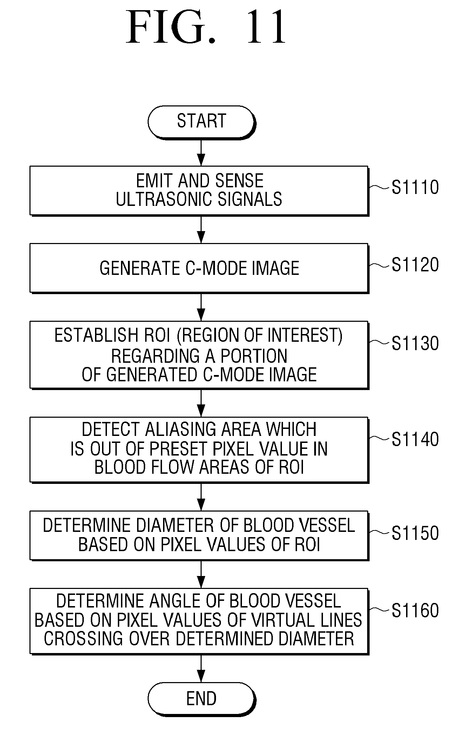

Referring to FIG. 11, the method of providing the blood vessel analysis information may include emitting and sensing an ultrasonic signal at operation S1110, generating a color mode image at operation S1120 and setting an ROI regarding one or more portions of the generated color mode image at operation S1130. The method may further include detecting an aliasing area that is out of a preset pixel value on a blood flow area of an ROI at operation S1140, determining at least one diameter of the blood vessel based on pixel values of an ROI image at operation S1150, and determining at least one angle of the blood vessel based on one or more pixel values on virtual lines crossing over the corresponding diameter at operation S1160.

The operation S1130 of setting the ROI may include setting the ROI on a previously established position of the generated color mode image, and adjusting the ROI so that a center coordinate value of the ROI is approached to the center coordinate value of a blood flow when the center coordinate value of the ROI is out of a previously established range from the center coordinate value of the blood flow in the generated color mode image.

Further, the method of providing the blood vessel analysis information may be performed on the ROI established corresponding to a portion of the generated color mode image. When image processing regarding the ROI completes, a next ROI may be newly established based on the determined angle of the blood vessel.

Further, the operation S1140 of detecting the aliasing area may include detecting the aliasing area by additionally considering a histogram of the generated color mode image.

Further, the operation S1140 of detecting the aliasing area may include performing the labeling operation in each section of the generated color mode image according to pixel values of the generated color mode image, performing the clustering operation on the labeled sections based on pixel values of the generated color mode image, determining the aliasing area based on a result of the clustering operation, and interpolating the determined aliasing area. The aliasing area is adjusted or changed to or is replaced with an interpolated area having interpolated pixel values (data) to be displayed together with adjacent areas of the blood vessel on a screen of the display 130 when the controller 120 performs the interpolating operation.

The operation S1140 of determining the aliasing area may include determining the labeled section as the aliasing area when there are more than two clustering areas within one labeled section.

The present general inventive concept can also be embodied as computer-readable codes on a computer-readable medium. The computer-readable medium can include a computer-readable recording medium and a computer-readable transmission medium. The computer-readable recording medium is any data storage device that can store data as a program which can be thereafter read by a computer system. Examples of the computer-readable recording medium include a semiconductor memory, a read-only memory (ROM), a random-access memory (RAM), a USB memory, a memory card, a blue-ray disc, CD or DVD-ROMs, magnetic tapes, floppy disks, and optical data storage devices. The computer-readable recording medium can also be distributed over network coupled computer systems so that the computer-readable code is stored and executed in a distributed fashion. The computer-readable transmission medium can transmit carrier waves or signals (e.g., wired or wireless data transmission through the Internet). Also, functional programs, codes, and code segments to accomplish the present general inventive concept can be easily construed by programmers skilled in the art to which the present general inventive concept pertains.

Further, the above described method of providing the blood vessel analysis information may be architected in embedded software type or firmware type, and provided in any one of a CISC (complex instruction set computer) chip, an RISC (reduced instruction set computer) chip, and a bit slice MPU (micro processing unit).

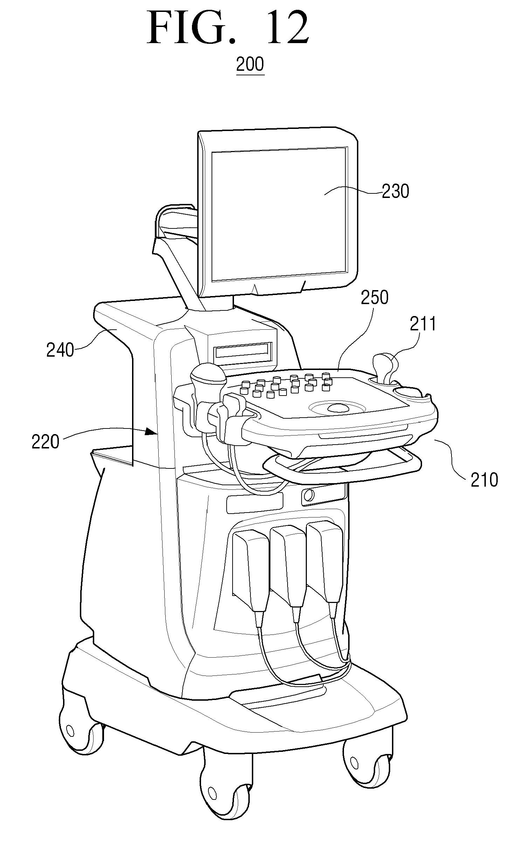

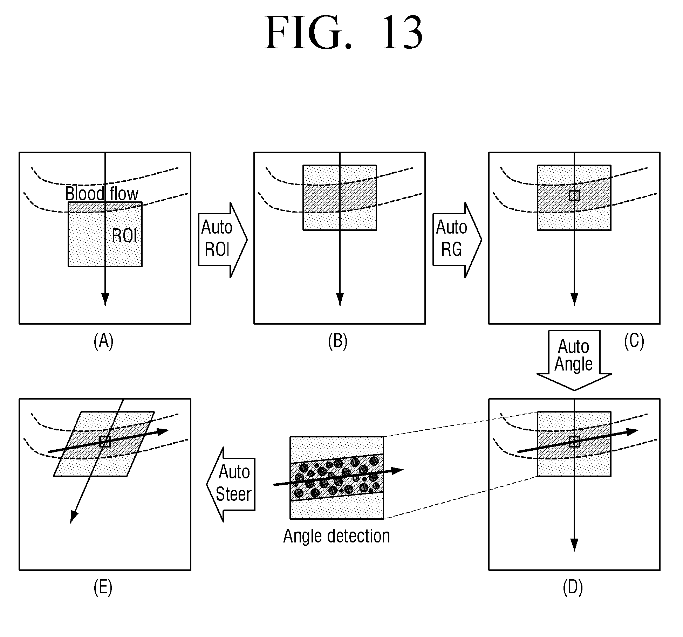

The blood vessel analysis information providing apparatus 100 may be implemented to be a pulse wave Doppler ultrasonic diagnostic apparatus. FIG. 12 illustrates a diagram of an ultrasonic diagnostic apparatus 200 according to an embodiment of the present general inventive concept, and FIG. 13 illustrates ultrasonic diagnosis by using the ultrasonic diagnostic apparatus 200 of FIG. 12.

The ultrasonic diagnostic apparatus 200 may include an ultrasonic transmitter and receiver 210 including a probe 211, a controller 220, a display 230, and a body; it may also include other additional units. Thus, it may further include a key input unit including various keys for various user settings in the ultrasonic diagnosis, and other various user interfaces 250. The ultrasonic transmitter and receiver 210, the controller 220, and the display 230 of the ultrasonic diagnostic apparatus 200 may correspond to the ultrasonic transmitter and receiver 110, the controller 120, and the display 130 of the blood vessel analysis information providing apparatus 100 illustrated in FIG. 1.

A user, for example, a diagnostician or medical doctor, may contact the probe 211 of the ultrasonic diagnostic apparatus 200 on a body portion to obtain blood vessel analysis information and drive the ultrasonic diagnostic apparatus 200. According to a setting operation performed by a user with a user input, at least one or a combination of a B-mode image and a C-mode image is generated and displayed on the display 230. The ultrasonic diagnostic apparatus 200 may have a unit to support a Doppler spectrum image. In this case, the display 230 displays the Doppler spectrum image as well as the B-mode or C-mode image.

The controller 220 may set an ROI regarding one or more portions of the generated color mode image. As described above, ROI indicates a body part for the purpose of emitting ultrasonic waves.

The controller 220 may set an ROI in the generated color mode image from a position of a body portion in which the diagnostician performs an initial operation to control or contact the probe. Further, the controller 220 determines whether a center coordinate value of an ROI is out of a preset range from center coordinate values of a blood flow in the generated color mode image. If the center coordinate value of the ROI is within the preset range, or similar to the center coordinate value of the blood flow, image processing may be performed. However, when a diagnostician controls the probe to be randomly disposed on a body portion and a center position of the probe is out of a blood vessel as illustrated in a viewA of FIG. 13, a center coordinate value of an ROI may be out of a preset range from the center coordinate value of the blood flow in the generated color mode image. In this case, the controller 220 adjusts the ROI according to an automatic ROI adjusting operation as illustrated in views A, B, and C of FIG. 13 so that a center coordinate value is approached to the center coordinate value of the blood flow as illustrated in a view C of FIG. 13.

However, as explained in the embodiment of the above described blood vessel analysis information providing apparatus 100, characteristics of ROIs can be different from each other according to clinical determining standards or preferences with respect to medical disease treatments. Further, when a signal to finish the diagnosis on an ROI is inputted automatically or by a user, a next ROI may be automatically established according to the above same method.

The ultrasonic diagnostic apparatus 200 may measure one or more diameters and one or more angles of a corresponding blood vessel regarding an ROI based on one or more pixel values of a color mode image likewise in the blood vessel analysis information providing apparatus 100 as illustrated in a view D of FIG. 13.

The controller 220 determines the diameter of the blood vessel based on the pixel values of the color mode image. The controller 220 may also consider the generated C-mode image and B-mode image together. In this case, a new image is generated by combining the B-mode image and the C-mode image. Although an anatomical boundary of a blood vessel and a surrounding organ cell can be observed in the B-mode image, a boundary may be difficult to be viewed by a user due to at least a noise. Although a relative velocity of a blood flow can be observed or detected in the C-mode image, color information may be insufficient to show (or display) the boundary to a user. According to an embodiment, noise that may occur in the B-mode and C-mode images can be minimized, by using a reconstituted image, and blood vessel information can be estimated and generated. The angle and diameter may be informed to a user with respect to an ROI using a visual image displayed on a display or an audio signal generated from an audio unit or speaker (not illustrated) of the ultrasonic diagnostic apparatus 200 according to a user design or preference.

Newly reconstituted image information may be calculated according to a following mathematical formula.

$$I_{f\text{-image}}(i,j) = \alpha * N_{b\text{-image}}(i,j) + \beta * N_{c\text{-image}}(i,j)$$

where $I_{f\text{-image}}$ is an reconstituted image (fusion image),

$N_{b\text{-image}}$ and $N_{c\text{-image}}$ are normalized B-mode and C-mode images,

$\alpha$ and $\beta$ are a weight factor, and

(i , j) is a pixel position where an image size is m * n ($0 \le i \le m$, $0 \le j \le n$).

For more efficient calculation, image reconstituting may be performed only on an ROI which will be described below.

The controller 220 may determine a width (distance or gap) of a blood vessel on lines having a shortest profile value of a blood flow area as a diameter of the blood vessel by comparing one or more profile values of the blood flow area regarding virtual lines crossing over one or more random pixels of the blood vessel in the generated color mode image. Herein, the one or more profile values of the blood flow area indicate a value generated by adding values of consecutive pixels which indicate the blood flow area.

In the C-mode image or the reconstituted image, one or more pixels values on a plurality of virtual lines crossing over a blood vessel may distinguish a blood flow area and other areas based on a preset threshold value. The blood flow area may be expressed in different values from other areas because of a blood flow rate. Thus, the profile values may be constituted based on these pixel values. At this process, the shortest line on one area whose profile values correspond to a blood flow area may be determined as a traverse line disposed orthogonal to the blood vessel. Further, a length of the profile value area becomes a diameter of the blood vessel.

Further, the controller 220 may determine one or more angles of a blood vessel. The controller 220 determines the angles of the blood vessel based on one or more pixel values on a plurality of virtual lines crossing over the determined diameter. At this process, a plurality of virtual lines crossing over the diameter may be considered in determining or calculating the angle. The above virtual lines are vectors of the blood vessel, and angles of the virtual lines from a horizontal plane become angles of the blood vessel. A plurality of virtual lines crossing over a diameter of a blood vessel can be considered. If the number of virtual lines is $N_{VR}$, $N_{VR}$ and the determined diameter $\Delta v$ satisfies a following relation.

$$N_{VR} \propto \Delta v / \lambda$$

where $\lambda$ is a width between a plurality of virtual lines.

Thus, the number of virtual lines is inverse proportional to the width of the virtual lines.

A plurality of virtual lines having various angles crossing over the diameter may be considered. Herein, a width of each virtual line may be generated by having a width of k° from -180° to 180°. As a k value is smaller, a resolution of the calculated angle may be greater; however, a too much small k value deteriorates a calculating speed. Herein, k may be used to be 1°, and can be managed (or set) automatically or manually according to performance of detecting angles.

At this process, the controller 220 may add pixel values regarding a plurality of virtual lines crossing over the determined diameter according to a sliding angle from the horizontal plane. It may add pixel values of virtual lines having the same angle and crossing over the diameter. Further, the controller 220 may determine an angle of a blood vessel based on sliding angles regarding a plurality of virtual lines and the smallest value among the added values of the pixel values. For example, if a result of adding the pixel values on virtual lines crossing over the diameter and having a small angle from the horizontal plane is less than a result of adding the pixel values on virtual lines crossing over the diameter and having different angles as illustrated in FIG. 4A, an angle corresponding to the former can be an angle of the blood vessel.

A representative value can be defined by mathematically formulizing the above. Thus, the controller 220 may calculate a representative value regarding a plurality of virtual lines crossing over the determined diameter according to sliding angles from the horizontal plane. A representative value is defined by a following mathematical formula.

$$E(\theta) = \Sigma^{NVR}_{i=1}\left(L_{i\_AVE} + k^* L_{i\_SD}\right) \ (-180 \leq \theta \leq 180)$$

where $E(\theta)$ is a representative value regarding a plurality of virtual lines having a sliding angle from the horizontal plane, $\theta$,

$N_{VR}$ is the number of virtual lines having a sliding angle from the horizontal plane, $\theta$,

$L_{i\_AVE}$ is an average value of pixel values on a virtual line at an i position having a sliding angle from the horizontal plane, $\theta$, and

$L_{i\_SD}$ is a standard deviation of pixel values on a virtual line at an i position having a sliding angle from the horizontal plane, $\theta$.

Further, the controller 220 determines each sliding angle from the horizontal plane regarding a plurality of virtual lines and determines the sliding angle of the virtual line having the smallest calculated representative value as an angle of the blood vessel.

The ultrasonic diagnostic apparatus 200 according to an embodiment may calculate diameters and angles of blood vessels according to the above described method as illustrated in a view E of FIG. 13.

Further, the ultrasonic diagnostic apparatus 200 may detect and interpolate one or more aliasing areas.

As explained above, an aliasing area indicates an area where there are pixel values more than a critical value of a blood flow rate in a blood flow area. An aliasing area may appear when a blocking section is viewed due to an exterior material of a blood vessel or when noise occurs due to other reasons. An aliasing area is a phenomenon in which pixel values are shifted to a certain level from a normal level due to a bias, not a value of a blood flow rate.

The controller 220 of the ultrasonic diagnostic apparatus 200 performs the labeling operation on each section regarding the color mode image according to pixel values of the generated color mode image. The controller 220 performs the clustering operation regarding the labeled section based on pixel values of the color mode image. Further, an aliasing area is determined based on a result of the clustering operation. At this process, when there are more than two clustering areas within one labeled section, the labeled section may be determined as an aliasing area. The controller 220 interpolates the determined aliasing area using pixel values of one or more adjacent areas.

Meanwhile, a blood vessel is usually connected (extended) to have a consecutive vessel shape, not an independent spot shape. Thus, a small spot size of a label is considered to be noise and removed. The labels remaining after noise components are removed are all the components constituting a blood vessel. Velocity distribution is calculated by performing the clustering operation based on color information (blood flow rate) in each label.

Further, the controller 220 controls the display 230 to display blood vessel information in which an aliasing area is removed.

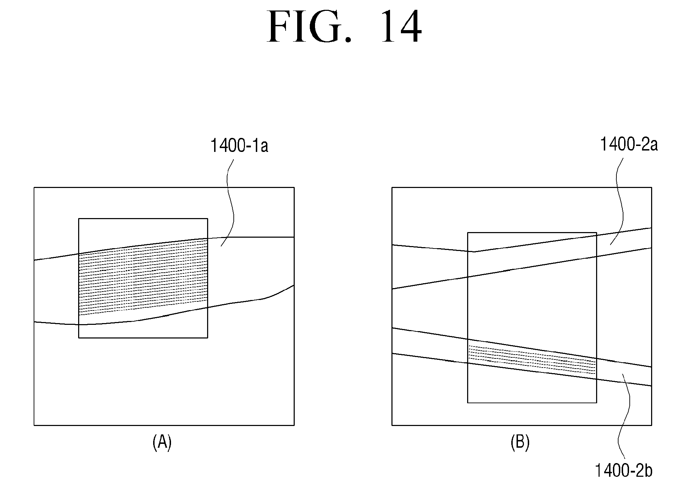

FIG. 14 is a diagram illustrating detection of a diameter and angle of a blood vessel by using the ultrasonic diagnostic apparatus of FIG. 12.

A view A of FIG. 14 illustrates a screen in which a diameter and an angle of a blood vessel are detected through the ultrasonic diagnostic apparatus 200. A view B of FIG. 14 illustrates a result of a detecting operation to detect a diameter and an angle by selecting one blood vessel when there are more than two blood vessels. Thus, according to an embodiment, a diameter and an angle of a blood vessel can be precisely detected on an ROI even when there are a plurality of blood vessels.

A blood vessel angiographic image apparatus will be explained hereinafter.

A blood vessel intervention is a medical activity to make a small hole on a skin portion, insert a catheter or a medical induced steel wire directly through the small hole of the skin portion, and observe one or more blood vessels with the image display apparatus, and perform the corresponding action, which is usable to affect various blood vessel related diseases. When the intervention is performed, a practitioner performs a corresponding operation while observing a blood vessel within a body at real time. Generally, observing blood vessels with a non-invasive method is called as angiography. The angiography is usable to check progression before and after diagnosis and treatment in addition to an operation of the intervention.

MRI, CT, ultrasound and X-ray may be usable for a method for the angiography to observe blood vessels within a body. Each method may have one or more different characteristics from other methods. The MRI and CT are used for a precise analysis of a blood vessel, and the X-ray and ultrasound are used for the intervention and observance on a blood vessel at real time.

When the intervention is performed, the angiography is used by injecting a contrast agent into a blood vessel within a body and photographing one or more X-ray images. The blood vessel may not be observed or determined from an image using a conventional X-ray photographing method; however, when the contrast agent is injected into a blood vessel, a clear blood vessel image may be obtained.

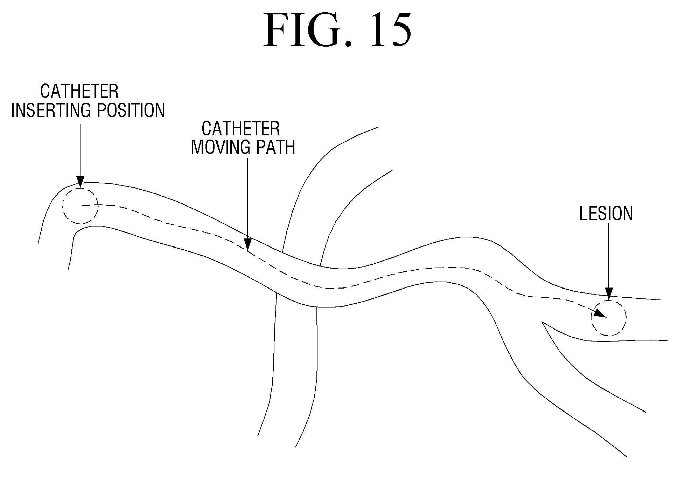

FIG. 15 illustrates a moving path from a top-casting position of a catheter.

As illustrated in FIG. 15, when performing intervention to use X-ray angiography, a user, for example, a medical practitioner such as clinician, controls or moves the catheter from an initially inserted position to a lesion position while viewing a two-dimensional X-ray image at real time. The users may control or move the catheter at different times according to experiences and skills of medical practitioners. As a time to move the catheter is taken longer, an operating time is taken longer. Further, an exposure time of radioactive rays is taken longer. Moreover, it may be required to additionally inject the contrast agents. When the catheter is quickly moved at a fast speed, it may be possible to damage a wall of a blood vessel due to the movement of the catheter.

The blood vessel angiographic image providing apparatus is provided to detect and recognize one or more blood vessels in the image, track the recognized blood vessels, determine diameters, angles, crossovers, and bifurcations regarding the blood vessels, and provide the images thereof when performing the intervention to use blood vessel information with the X-ray. Further, moving paths from the inserting position of the catheter to the lesion position may be determined by using the obtained blood vessel information.

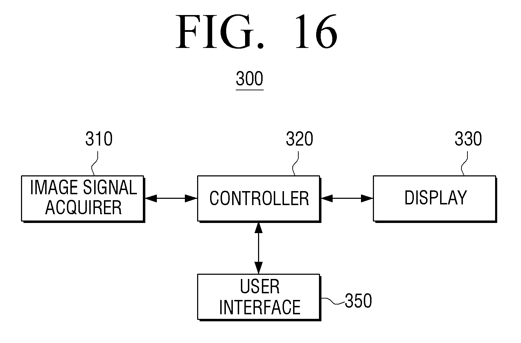

FIG. 16 is a block diagram illustrating a blood vessel angiographic image apparatus 300 according to an embodiment of the present general inventive concept.

Referring to FIG. 16, the blood vessel angiographic image apparatus 300 according to an embodiment includes an image signal acquirer 310, a controller 320, a display 330, and a user interface 350. The controller 320 and the display 330 may be similar to the controller 120 and the display 130 of the blood vessel analysis information providing apparatus 100 of FIG. 1, and the user interface 350 may be similar to the user interface 250 of the ultrasonic diagnostic apparatus 200 of FIG. 12. In this case, the blood vessel angiographic image apparatus 300 may sense an ultrasonic echo signal reflected from a body portion by emitting an ultrasonic signal on a body where a blood vessel is disposed, constitute (generate) an image, and display the image on the display 330. However, the blood vessel angiographic image apparatus 300 may include other units different form the above described units and may display an image according to at least one of different methods. For example, it may display a two-dimensional X-ray image.

A user (operator) establishes an inserting position of a catheter and a lesion position when viewing a blood vessel angiographic image displayed on the display 330. The two positions established by a user become a start and an end of a moving path of the catheter.

The above setting can be performed with the user interface 350. The setting method may be implemented to be various types such as clicking a screen with a mouse and directly touching the screen with a finger or a pen unit.

The established two positions may be expressed in a format which can be visually recognized in the displayed blood vessel angiographic image. For example, a moving path of the catheter may be highlighted and displayed so as to be more clearly visible in FIG. 15.

A clinician inserts the catheter on the position near to the lesion position and injects a contrast agent. Further, when the blood vessel is clearly viewed from the blood vessel angiographic image, a clinician views the image, moves the catheter to the lesion position, and continues to make an operation.

When the contrast agent is injected on a specific position of a blood vessel, it flows through blood vessels because of blood circulation. Such a contrast agent has characteristics to block an X-ray. Thus, the blood vessels are expressed more clearly in an image. The contrast agent performs its role when staying in the blood vessels according to a circulation speed of the blood. As time goes, it disappears from the blood vessels.

The blood vessel angiographic image apparatus 300 generates and/or outputs a diameter, an angle, a crossover and a bifurcation of a blood vessel as an image by using an image processing technology. The bifurcation indicates a point in which one blood vessel flows (is divided) into two blood vessels, and the crossover indicates a point in which two blood vessels disposed on different planes are crossed from a view of a two-dimensional image.

The controller 320 analyzes blood vessel information.

Thus, the controller 320 measures a diameter and an angle of a blood vessel using, for example, a virtual ray and/or the above described method. It may use an image of a reconstituting B-mode and/or C-mode images by using the ultrasonic image; however, a single mono image can be used.

In this mono image, the controller 320 may determine a diameter of a blood vessel on lines having the shortest profile value of a blood flow area as a diameter of a blood vessel by comparing profile values of a blood flow area regarding virtual lines crossing over random pixels of a blood vessel. Herein, the profile values of the blood flow area indicate a value obtained by adding values of consecutive pixel values which indicate the blood flow area.

In the mono image, pixel values on a plurality of virtual lines crossing over a blood vessel may distinguish a blood flow area and other areas (for example, non-blood flow area or non-blood vessel area) based on a preset threshold. A blood flow area can be expressed in different values from values of other areas because of a blood flow rate. Therefore, profile values may be generated based on the pixel values. At this process, a shortest line on an area whose profile values correspond to a blood flow area may be determined as a traverse line disposed orthogonal to the blood vessel. Further, a length of the profile value area having the shortest line becomes a diameter of the blood vessel.

When an ROI is set, a diameter of a blood vessel ($\Delta v$) are measured in a range gate which is a center coordinate of a blood flow in the set ROI.

Next, the controller 320 determines one or more angles of a blood vessel. The controller 320 determines an angle of the blood vessel based on one or more pixel values on virtual lines crossing over the determined diameter. As illustrated in a view A of FIG. 4, virtual lines 41 crossing over the determined diameter may be considered to determine an angle of the blood vessel. The angle of the blood vessel may indicate a direction, in which the blood vessel is extended or the blood flows in the blood vessel, with respect to a reference line or plane, for example, a horizontal line or plane of a screen of the display, The angles may be changed according to the direction of the blood vessel or a curvature of the blood vessel. An angle of an ROI may be same as an angle of a next ROI. It is possible that an angle of an ROI may be different from an angle of a next ROI. If there are virtual lines passing through a center of a blood vessel, the virtual lines may be vectors of the blood vessel, and an angle between the virtual lines and the horizontal plane may be an angle of the blood vessel. The virtual lines may be distinguishable.

At this process, as illustrated in a view A of FIG. 4, it may consider a plurality of virtual lines 41 crossing over the determined diameter. If the number of virtual lines is $N_{VR}$, $N_{VR}$ and the determined diameter $\Delta v$ satisfies a following relation.

$$N_{VR} \propto \Delta v / \lambda$$

where $\lambda$ is a width between a plurality of virtual lines.

Thus, the number of virtual lines is inversely proportional to the width of the virtual lines.

A plurality of virtual lines having various angles crossing over the diameter may be considered. A view B of FIG. 4 illustrates the above described lines 43. Herein, the width of each virtual line may be generated in a range of the width of k° from -180° to 180°. As a value k is smaller, a resolution of the calculated angle may be greater; however, a too much small value k deteriorates a calculating speed. Herein, a value k may be 1°, and can be set, controlled, generated, or managed automatically or manually according to performance and/or efficiency of an angle detecting operation.

At this process, the controller 320 may add pixel values regarding a plurality of virtual lines crossing over the determined diameter according to a sliding angle from the horizontal plane. In a view A of FIG. 4, it may add pixel values of virtual lines having the same angle and crossing over the diameter. Further, the controller 320 may determine an angle of the blood vessel based on sliding angles regarding a plurality of virtual lines having the smallest value among the added values of pixel values. For example, if a result of the adding operation of adding pixel values on virtual lines crossing over the diameter and having a small angle from the horizontal plane is less than a result of the adding operation of adding pixel values on virtual lines crossing over the diameter and having different angles in a view A of FIG. 4, an angle corresponding to the former can be an angle of the blood vessel.

A representative value can be defined by mathematically formulizing the above. Thus, the controller 320 may calculate a representative value regarding a plurality of virtual lines crossing over the determined diameter according to sliding angles from the horizontal plane. A representative value is defined by a following mathematical formula.

$$E(\theta) = \sum\nolimits_{i=1}^{NVR}(L_{i\_AVE} + k^* L_{i\_SD}) \ (-180 \leq \theta \leq 180)$$

where $E(\theta)$ is a representative value regarding a plurality of virtual lines having a sliding angle $(\theta)$ from a horizontal plane,

$N_{VR}$ is the number of virtual lines having a sliding angle $(\theta)$ from the horizontal plane,

$L_{i-AVE}$ is an average value of pixel values on a virtual line at an i position having a sliding angle $(\theta)$ from the horizontal plane, and

$L_{i\_SD}$ is a standard deviation of pixel values on a virtual line at an i position having a sliding angle $(\theta)$ from the horizontal plane.

Further, the controller 320 determines a sliding angle from the horizontal plane regarding a plurality of virtual lines having the smallest calculated representative value as an angle of the blood vessel.

$E(\theta)$ and $\Delta v$ usable to calculate a diameter and an angle of a blood vessel are applied to the above formula when determining crossovers and bifurcations. An average pixel value within a blood vessel is used when the crossovers and bifurcations are determined. The contrast agent flows with the blood in the blood vessel. Contrast (pixel values) of a blood vessel expressed in the blood vessel angiographic image becomes different according to a density of the contrast agent within the blood vessel. Thus, the blood vessel (e.g., bifurcated blood vessels) may have an average contrast having similar bandwidths, and other crossover blood vessels may have different contrast bandwidths. By using the above characteristics expressed by the pixels, bifurcations and crossovers are determined.

In a process of selecting a blood vessel at a bifurcation, a user determination is applied. When there are various paths from the bifurcation to a lesion position, the automatic method to select at least one of blood vessels can be performed. However, because it may be important to determine based on clinical standards such as moving easiness of the catheter according to a diameter and/or shape of a blood vessel, a selection can be made according to user clinical determination.

Moving information of the catheter is calculated based on the information obtained from automatic calculations and user determination.

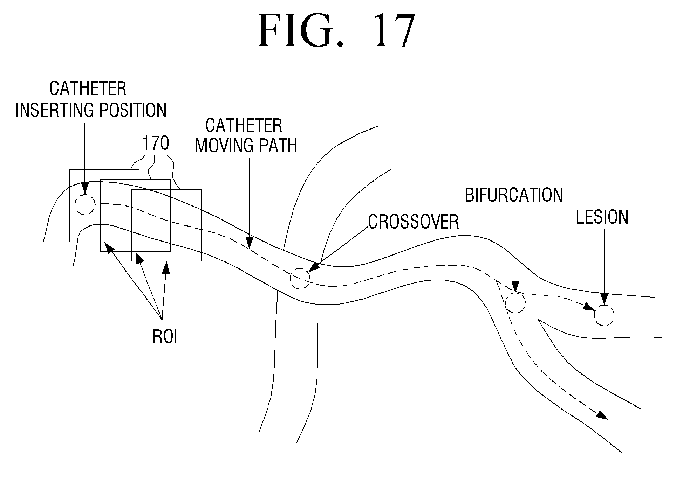

FIG. 17 illustrates a screen to display a moving path regarding a catheter according to an embodiment of the present general inventive concept.

As illustrated in FIG. 17, the display 330 of the blood vessel angiographic image apparatus 300 may display one or more moving paths of a catheter in a dotted line shape, and a user may modify at least one of the moving paths (e.g., selecting at least one of blood vessels at bifurcations). The path may be usable as information. Based on the information on the path, the medical activity can be performed in addition to performing the intervention. Further, information regarding an inserting position of the catheter, a lesion position, and the moving paths of the catheter are stored with positions of corresponding blood vessels, which may be used in various applications such as follow-ups after the medical treatment operation.

The information measured and calculated described above is expressed in the blood vessel angiographic image (e.g., the inserting position of the catheter, the lesion position, bifurcation and crossover information, the moving paths, and distances of the catheter), and is displayed (provided) in a proper shape to a user when performing the intervention and/or the medical treatment.

Meanwhile, the above determining diameters and angles of blood vessels may be performed only on an ROI 170 as illustrated in FIG. 17. ROI indicates a body portion where an ultrasonic signal is projected.

The controller 320 establishes an ROI on a preset position of a mono image. Further, the controller 320 determines whether a center coordinate value regarding an ROI is out of a center coordinate value regarding a blood flow in the generated color mode image. If the center coordinate value regarding the ROI is within a previously established range from or is similar to the center coordinate value regarding the blood flow, image processing may be performed. However, if the center coordinate value regarding the ROI is out of the previously established range from or is not similar to the center coordinate value regarding the blood flow in the generated color mode image, the controller performs an ROI position adjusting process to adjust the ROI from an original portion to a new position so that the center coordinate value regarding the ROI is approached to the center coordinate value regarding the blood flow.

ROI can be different according to clinical determining standards regarding diseases. Further, it can be different according to a health condition, age, food digesting status, body condition, and/or body portion regarding a patient.

In order to move on to a next ROI for image processing after completion of the image processing regarding the established ROI, the controller 320 may newly establish a new ROI based on the determined angle of the blood vessel described above. FIG. 17 illustrates a process to move an ROI from the established ROI to another ROI. ROI may be established by considering one or more angles of the blood vessel.

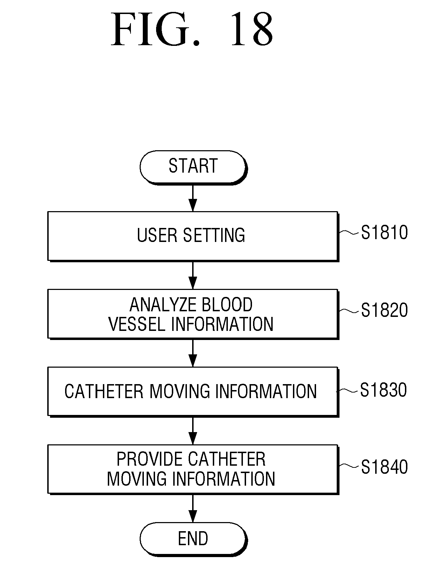

FIG. 18 is a flowchart illustrating a method of displaying a blood vessel angiographic image according to an embodiment of the present general inventive concept.

Referring to FIG. 18, the blood vessel angiographic image displaying method includes performing setting by a user using a user interface or a user input unit (one or more keys or menus of a panel) at operation S1810, analyzing blood vessel information at operation S1820, calculating moving information of a catheter at operation S1830, and providing the moving information of the catheter at operation S1840.

At operation S1810, an ultrasonic signal is outputted and an blood vessel angiographic image is displayed. A user checks the image and establishes an inserting position of the catheter and a lesion position. Also, at operation S1810, ultrasound can be usable to obtain an image signal. However, the present general inventive concept is not limited thereto. Another method using another medium than the ultrasound can be usable to obtain an image signal. The setting may include an input to control one or more characteristics of the image regarding the body portion including the blood vessel and also control information thereof to be displayed on a display as described above or hereinafter.

At operation S1820, the blood vessel angiographic image apparatus calculates one or more diameters and one or more angles of one or more blood vessels and determines one or more crossovers and one or more bifurcations.

At operation S1830, the moving information of the catheter is calculated and expressed so as to be distinguished. The moving information of the catheter is displayed on a screen of a display unit independently or together with the displayed image. The moving information may be superimposed, highlighted, or inserted into a portion of the displayed image. The moving information may be generated as sound using an audio unit (not illustrated)

At operation S1840, the finally measured and calculated information is displayed on the display.

An medical image registering apparatus will be explained in more detail hereinafter.

When a uniform scene or object is photographed with different equipment or different perspectives, objects viewed in each of the images have different shapes and coordinate systems from each other. An image registration is a method to express (or display) the different photographed objects on one uniform (common) coordinate system. The image registration is one of computer vision methods usable in a medical image field. Examples of the representative applications requesting the image registration are as follows.

The method may include comparing images regarding affected portions which change as time goes for the purpose of observing disease progression and comparing the images before and after an medical operation (a uniform diagnostic apparatus is used, e.g., CT-CT)

The method may include simultaneously requesting anatomical information and functional information on a specific spot (different types of diagnostic apparatuses are used, e.g., MRI-CT). The anatomical information may be obtained in a diagnostic apparatus using CT, X-ray, ultrasound, etc. The anatomical information may also be obtained in the diagnostic apparatus using MRI, fMRI, PET, SPECT. Etc.

The method may include enabling to perform a medical operation at real time (different types of diagnostic apparatuses, e.g., CT-ultrasound)

It is possible that the image registration can be usable for various purposes more than the above described method. The image registration searches for a uniform spot on the photographed objects from the two sub-images in different states; the uniform spot is called as a landmark of the registration. The medical images may include one or more landmarks such as bone information, blood vessel information, and an outer edge of an organ (tissue structure).

It may be easy to automatically detect such landmarks in CT or MRI images having a better resolution or image quality. However, since ultrasonic images have characteristics of a lower image quality and a lower SNR, it may not be easy to detect the landmarks. Further, since the ultrasonic images have characteristics in that the photographed body portion is local, differently from other diagnostic apparatuses, it may be difficult to observe a wider range of a body portion.

However, in the ultrasonic system, there is no exposure of radioactive rays, observing can be performed at real time, and a cost for the analysis is relatively lower. Thus, the ultrasonic apparatus is a useful apparatus among medical image apparatuses. Further, the above described operation, such as intervention, can be performed in the ultrasonic apparatus. Before the operation, planning may be performed by using a higher precise image such as a CT image; however, when performing the operation, the ultrasonic apparatus can enable a user to observe the body part through the image at real time.

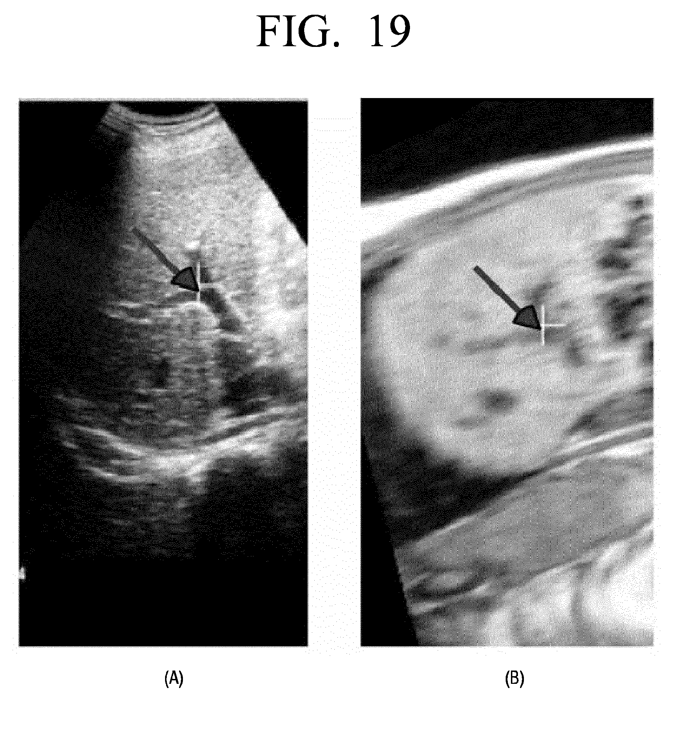

FIG. 19 illustrates an image photographed with different apparatuses regarding the same portion of a human body.

As illustrated in sub-images A and B of FIG. 19, image registration is performed to arrange objects viewed from the two sub-images in the same size and direction. Therefore, corresponding technology is provided to detect one or more landmarks applied when performing the image registration.

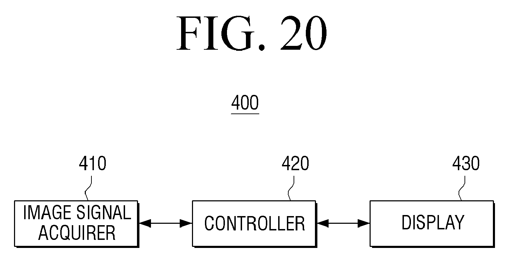

FIG. 20 is a block diagram illustrating a medical image registering apparatus 400 according to an embodiment of the present general inventive concept.

Referring to FIG. 20, the medical image registering apparatus 400 according to an embodiment includes an image signal acquirer 410, a controller 420 and a display 430. The controller 420 and the display 430 may be respectively similar to the controller 120 and the display 130 of the blood vessel analysis information providing apparatus 100 of FIG. 1. When the image signal acquirer 410 of the medical image registering apparatus 400 includes an ultrasonic transmitter and receiver, it may emit an ultrasonic signal on a body portion to correspond to a blood vessel and/or a blood flow, sense an ultrasonic echo signal reflected from the body portion, constitute (or generate) an image, and display the constituted image on the display 430. However, the image signal acquirer 410 may obtain an image using other different various methods than the above described method.

The blood vessel information may be obtained using the above described blood vessel analysis method. First, the controller 420 calculates one or more diameters and one or more angles of a blood vessel. The mentioned measuring method (using virtual lines) reconstitutes B-mode and/or C-mode images in an ultrasonic image to create a new image and uses corresponding image values to obtain the blood vessel information. However, a mono image regarding images of CT or MRI can be used. Further, the method detects one or more bifurcations where blood vessels are parted (divided or extended) and respectively measures one or more angles of corresponding bifurcations.

Thus, in this mono image, the controller 420 may determine a diameter (distance or width) of a blood vessel on one or more lines having the shortest profile value of a blood flow area as a diameter of the blood vessel by comparing one or more profile values of a blood flow area regarding one or more virtual lines crossing over at least one random pixel of the blood vessel. Herein, the profile values of the blood flow area indicate a value generated by adding values of consecutive pixel values which indicate the blood flow area.

In the mono image, pixel values on a plurality of virtual lines crossing over a blood vessel may distinguish an blood flow area and other areas based on a preset threshold. A blood flow area is expressed in different values compared to other areas because of a blood flow rate. Therefore, profile values may be constituted based on the pixel values. At this process, the shortest line on an area whose profile values correspond to a blood flow area may be determined as an area having a traverse line orthogonal to the blood vessel. Further, a length of the profile value area having the shortest line becomes a diameter of the blood vessel.

When an ROI is set, at least one diameter of blood vessel ($\Delta v$) may be measured in a range gate which is a center coordinate of a blood flow in the set ROI.

Next, the controller 420 determines one or more angles of a blood vessel. The controller 420 determines an angle of the blood vessel based on pixel values on corresponding virtual lines crossing over the determined diameter. As illustrated in a view A of FIG. 4, the virtual lines 41 crossing over the determined diameter may be considered. If there are virtual lines passing through a center of a blood vessel, the virtual lines may be vectors of the blood vessel, and one of the angles between the virtual lines and the horizontal plane may be an angle of the blood vessel. The objective is to distinguish these virtual lines.

At this process, as illustrated in a view A of FIG. 4, it may consider a plurality of virtual lines 41 crossing over the determined diameter. If the number of virtual lines is $N_{VR}$, $N_{VR}$ and the determined diameter $\Delta v$ satisfies a following relation.

$$N_{VR} \propto \Delta v / \lambda$$

where $\lambda$ is a width between a plurality of virtual lines.

Thus, the number of virtual lines is inversely proportional to the width of the virtual lines.

A plurality of virtual lines having various angles crossing over the diameter may be considered. A view B of FIG. 4 illustrates the above described virtual lines 43. Herein, a width of each virtual line may be generated by having a width of k° from -180° to 180°. As a value k is smaller, a resolution of the calculated angle may be greater; however, a too small value k deteriorates a calculating speed. Herein, k may be used to be 1°, and can be managed automatically or manually according to performance of an angle detecting operation.

At this process, the controller 420 may add pixel values regarding a plurality of virtual lines crossing over the determined diameter according to a sliding angle from the horizontal plane. Referring to a view A of FIG. 4, the controller 420 may add pixel values of virtual lines having the same angle and crossing over the diameter. Further, the controller 420 may determine an angle of the blood vessel based on sliding angles regarding a plurality of virtual lines, that is, determine an angle of one virtual line having the smallest value among the adding values of pixel values as the angle of the blood vessel. For example, if an adding result of one or more pixel values on a virtual line crossing over the diameter and having a small angle from the horizontal plane is less than an adding result of pixel values on virtual lines crossing over the diameter and having different angles from the horizontal plane as illustrated in a view A of FIG. 4, an angle corresponding to the former can be an angle of the blood vessel.

A representative value can be defined by mathematically formulizing the above process. Thus, the controller 420 may calculate a representative value regarding a plurality of virtual lines crossing over the determined diameter according to sliding angles from the horizontal plane. A representative value is defined by a following mathematical formula.

$$E(\theta) = \sum_{i=1}^{NVR}(L_{i\_AVE} + k^* L_{i\_SD}) \ (-180 \le \theta \le 180)$$

where $E(\theta)$ is a representative value regarding a plurality of virtual lines having a sliding angle ($\theta$) from the horizontal plane,

$N_{VR}$ is the number of virtual lines having a sliding angle $\theta$ from the horizontal plane,

$L_{i\_AVE}$ is an average value of pixel values on a virtual line at i position having a sliding angle ($\theta$) from the horizontal plane, and

$L_{i\_SD}$ is a standard deviation of pixel values on a virtual line at i position having a sliding angle ($\theta$) from the horizontal plane.

Further, the controller 420 determines a sliding angle from the horizontal plane regarding a plurality of virtual lines having the smallest calculated representative value as an angle of the blood vessel.

Further, the controller 420 may detect a plurality of pieces of uniform information, similar information to be obtained according to the above process, by using another image in which the image registration is performed.

Next, the controller 420 detects feature values by using a plurality of pieces of blood vessel information obtained from each of the images. Because angles of bifurcations can be different according to angles of photographing images among the obtained blood vessel information, there may be slight differences between angles even regarding the same bifurcation. For the above process, a plurality of bifurcations are used.

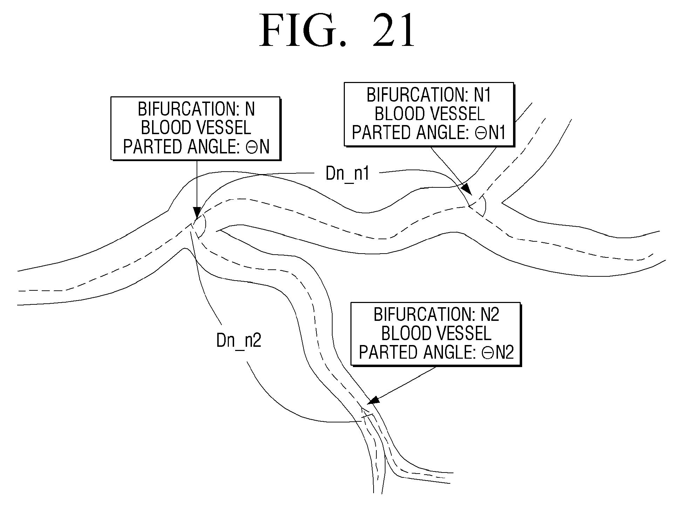

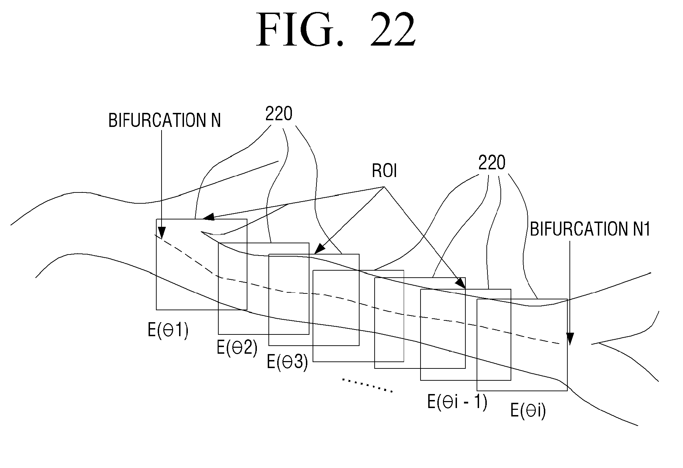

FIG. 21 illustrates angles of blood vessel bifurcations and distances between bifurcations, and FIG. 22 illustrates detecting one or more landmarks for the image registration.

Referring to FIG. 21, when a blood vessel is parted into two blood vessels, a bifurcation angle on a bifurcation at n times (or at n bifurcation position) may be $\theta$n. When the parted two blood vessels are parted again, bifurcation angles at this process may be respectively $\theta$n1 and $\theta$n2. The lengths (Dn_n1, Dn_n2) of blood vessels between bifurcations define that lengths of blood vessels from the bifurcation n to parted bifurcations n1, n2 are respectively Dn_n1 and Dn_n2. Herein, lengths of blood vessels indicate distances of curves regarding blood vessels when the blood vessel is in a curved shape.

The angle vectors (Vn_n1, Vn_n2) of blood vessels between bifurcations detect angle vectors of blood vessels from a bifurcation n to next bifurcations n1 and n2. Such angle vectors may be obtained by measuring angles of blood vessels while moving an ROI when angles of blood vessels are measured according to the described in this specification.

$$Vn\_n1 = [E(\theta1)\ E(\theta2)\ E(\theta3)\ \cdots\ E(\theta i\text{-}2)\ E(\theta i\text{-}1)\ E(\theta i)]$$

Herein, i is the number of ROIs which are measured. Each feature value described above is performed on every bifurcation viewed from the image. The finally obtained feature values from one image are a bifurcation angle ($\theta n$) at one bifurcation, bifurcation angles ($\theta n1$, $\theta n2$) of the parted blood vessels at a bifurcation n, lengths ($Dn\_n1$, $Dn\_n2$) of blood vessels from a bifurcation n to bifurcations n1 and n2, length ratios ($Dn\_n1 / Dn\_n2$) of the two parted blood vessels, and angle vectors ($Vn\_n1$, $Vn\_n2$) of blood vessels between bifurcations. At least one of the parted two blood vessels can be selected for the medical treatment or for the moving path of the catheter automatically or manually. The ROI may be generated and displayed along the selected blood vessel.

Through the above processes, each feature value are estimated and calculated from one or two images applied in the image registration. The uniform spot viewed from the two images is searched by using corresponding feature values in a mono image or in a combination image. The corresponding spot is used as a landmark when performing the image registration.

Meanwhile, the above described detecting landmarks may be performed based on one or more ROIs 220 while moving from one bifurcation to next bifurcation, as illustrated in FIG. 22. ROI indicates a body portion where an ultrasonic signal is projected.

The controller 420 establishes an ROI on a preset position of the mono image. Further, the controller 420 determines whether a center coordinate value regarding the ROI is out of a center coordinate value regarding a blood flow in a generated color mode image. If center coordinate value regarding the ROI is within a previously established range from or similar to the center coordinate value regarding the blood flow, image processing may be performed. However, if the center coordinate value regarding the ROI is out of the previously established position from the center coordinate value regarding the blood flow in the generated color mode image, the ROI is adjusted so that the center coordinate value regarding ROI is approached to the center coordinate value regarding the blood flow.

ROI can be different from other ROI according to clinical determining standards regarding diseases. Further, it can be different according to a health condition, age, food digesting status, body condition, and/or body position regarding a patient.

In order to move on to the next ROI for image processing upon completion of the image processing regarding the established ROI, the controller 420 may newly establish a new ROI based on the determined angles of blood vessels described above. FIG. 22 illustrates moving of ROI 220. ROI may be established by considering angles of blood vessels.

The following will explain a method of designating an image registration landmark according to an embodiment of the present general inventive concept.

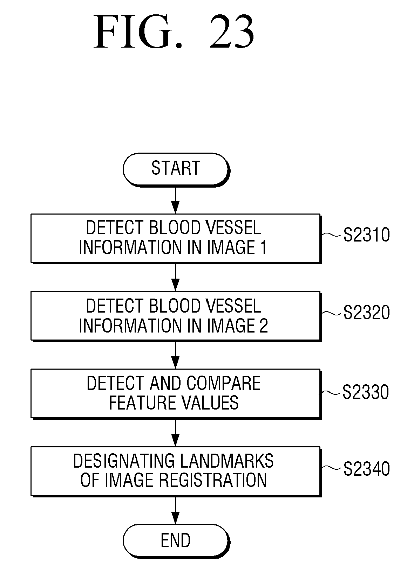

Referring to FIG. 23, the image registration landmark designating method according to an embodiment may include detecting blood vessel information in an image 1 at operation S2310, detecting blood vessel information in an image 2 at operation S2320, detecting and comparing feature values at operation S2330 and designating one or more image registration landmarks at operation S2340. Since the above operations have been described above, detail descriptions thereof will be omitted.

Although a few embodiments of the present general inventive concept have been shown and described, it will be appreciated by those skilled in the art that changes may be made in these embodiments without departing from the principles of the general inventive concept, the scope of which is defined in the appended claims and their equivalents.

**Claims**

1.   A method of providing blood vessel analysis information, the method comprising:

>  emitting an ultrasonic signal on a body portion corresponding to a blood vessel flow and sensing a reflected ultrasonic signal;
>  generating a color mode image by using the sensed ultrasonic signal; and
>  determining a diameter of the blood vessel based on one or more pixel values of the generated color mode image.

2.   The method of claim 1, wherein the determining the diameter of blood vessel comprises determining a shortest blood flow area to be the diameter of the blood vessel, by comparing one or more profile values regarding the pixel values on virtual lines crossing over the blood vessel in the generated color mode image.

3.   The method of claim 1 or 2, further comprising:

determining an angle of the blood vessel based on the pixel values on virtual lines crossing over the determined diameter.

4. The method of claim 3, wherein the determining the angle of the blood vessel comprises:

adding pixel values on the virtual lines crossing over the determined diameter according to a sliding angle from a horizontal plane; and
determining the angle of the blood vessel based on sliding angles regarding the virtual lines having the smallest value of the added pixel values.

5. The method of claim 3, wherein the determining the angle of the blood vessel comprises:

calculating a representative value regarding of the virtual lines crossing over the determined diameter according to a sliding angle from a horizontal plane; and
determining the angle of the blood vessel based on sliding angles regarding the virtual line and the smallest calculated representative value,
wherein the representative value is calculated according to a following mathematical formula:

$$E(\theta) = \sum_{i=1}^{N_{VR}}(L_{i\_AVE} + \kappa * L_{i\_SD}), \quad (-180 \leq \theta \leq 180)$$

where $E(\theta)$ denotes a representative value regarding a plurality of virtual lines having a sliding angle $(\theta)$ from the horizontal plane,
$N_{VR}$ is the number of virtual lines having a sliding angle $(\theta)$ from the horizontal plane,
$L_{i\_AVE}$ is an average value of pixel values on a virtual line at i position having a sliding angle $(\theta)$ from the horizontal plane, and
$L_{i\_SD}$ is a standard deviation of pixel values on a virtual line at i position having a sliding angle $(\theta)$ from the horizontal plane.

6. The method of any of claims 1 to 5, further comprising:

establishing a region of interest (ROI) regarding a portion on the generated color mode image,
wherein the ROI comprises an image regarding a center of the blood vessel, and the method is performed regarding the ROI.

7. The method of claim 6, the establishing ROI, comprising:

establishing the ROI on a previously established position of the generated color mode image; and
adjusting the ROI so that a center coordinate of the ROI is approached to a center coordinate of a blood flow of the blood vessel when the center coordinate of the ROI is out of a preset range from the center coordinate of blood flow in the generated color mode image.

8. The method of claim 3, wherein the method is performed on an ROI established regarding a portion of the generated color mode image, and a new ROI is newly established based on the determined angle of the blood vessel when image processing regarding the ROI completes.

9. The method of any of claims 1 to 8, further comprising:

distinguishing a blood flow area based on the pixel values of the generated color mode image, and detecting an aliasing area whose pixels values are out of a preset pixel value on the distinguished blood flow area.

10. The method of claim 9, wherein the detecting the aliasing area comprises detecting the aliasing area by further considering a histogram of the generated color mode image.

11. The method of claim 9, wherein the detecting the aliasing area comprises:

performing labelling in each section of the generated color mode image according to the pixel values of the generated color mode image;
performing clustering regarding the labelled sections based on the pixel values of the generated color mode image;
determining the aliasing area based on the clustering results; and
interpolating the determined aliasing area.

12. The method of claim 11, wherein the determining the aliasing area comprises determining the labelled section to be the aliasing area when there are more than two clustering areas within one labelled section.

13. An apparatus (100) to provide blood vessel analysis information, the apparatus comprising:

a display (130);
an ultrasonic transmitter and receiver (110) configured to emit an ultrasonic signal on an object and sense a reflected ultrasonic signal; and
a controller (120) configured to generate a color mode image by using the sensed ultrasonic signal and determine a diameter of a blood vessel based on one or more pixel values of the generated color mode image.

14. The apparatus (100) of claim 13, wherein the controller (120) determines a shortest blood flow area as the diameter of the blood vessel, by comparing one or more profile values regarding the pixel values on virtual lines crossing over the blood vessel in the generated color mode image.

15. The apparatus (100) of claim 13 or 14, wherein the controller (120) determines an angle of the blood vessel based on the pixel values of the virtual lines crossing over the determined diameter.

# FIG. 1

100

110

| ULTRASONIC TRANSMITTER AND RECEIVER | CONTROLLER | DISPLAY |

120 130

# FIG. 2

# FIG. 3

B-mode 31

C-mode 33

Fusion

Fusion Image 35

Measure

RD 37
D Vessel diameter

Auto Angle

Angle detection with Virtual rays

39
RH
Aa
Ar
D
RV

# FIG. 4

(A)

(B)

# FIG. 5

(A)　　　　　　　(B)　　　　　　　(C)

# FIG. 6

# FIG. 7

SAME
BLOOD VESSELS   ALIASING

FIG. 8

# FIG. 9

```
          ┌──────────────┐
          │    START     │
          └──────────────┘
                 │
                 ▼
   ┌─────────────────────────┐
   │     EMIT AND SENSE      │    S910
   │   ULTRASONIC SIGNALS    │
   └─────────────────────────┘
                 │
                 ▼
   ┌─────────────────────────┐
   │  GENERATE C-MODE IMAGE  │    S920
   └─────────────────────────┘
                 │
                 ▼
   ┌─────────────────────────┐
   │ DETERMINE DIAMETER OF BLOOD │   S930
   │ VESSEL BASED ON PIXEL VALUES OF │
   │ GENERATED COLOR MODE IMAGE │
   └─────────────────────────┘
                 │
                 ▼
          ┌──────────────┐
          │     END      │
          └──────────────┘
```

# FIG. 10

START

EMIT AND SENSE
ULTRASONIC SIGNALS — S1010

GENERATE C-MODE IMAGE — S1020

DETERMINE DIAMETER OF BLOOD
VESSELS BASED ON PIXEL VALUES — S1030
OF GENERATED C-MODE IMAGE

DETERMINE ANGLE OF BLOOD
VESSEL BASED ON PIXEL VALUES — S1040
ON VIRTUAL LINES CROSSING
OVER DETERMINED DIAMETER

END

# FIG. 11

START

EMIT AND SENSE
ULTRASONIC SIGNALS — S1110

GENERATE C-MODE IMAGE — S1120

ESTABLISH ROI (REGION OF INTEREST)
REGARDING A PORTION
OF GENERATED C-MODE IMAGE — S1130

DETECT ALIASING AREA WHICH
IS OUT OF PRESET PIXEL VALUE IN
BLOOD FLOW AREAS OF ROI — S1140

DETERMINE DIAMETER OF BLOOD VESSEL
BASED ON PIXEL VALUES OF ROI — S1150

DETERMINE ANGLE OF BLOOD VESSEL
BASED ON PIXEL VALUES OF VIRTUAL LINES
CROSSING OVER DETERMINED DIAMETER — S1160

END

# FIG. 12

200

# FIG. 13

(A)

Auto
ROI

(B)

Auto
RG

(C)

Auto
Angle

Angle detection

(D)

Auto
Steer

(E)

# FIG. 14

1400-1a

1400-2a

(A)

(B)

1400-2b

# FIG. 15

CATHETER
INSERTING POSITION

CATHETER
MOVING PATH

LESION

# FIG. 16

300

310                 320                 330

| IMAGE SIGNAL ACQUIRER | ◄──► | CONTROLLER | ◄──► | DISPLAY |

CONTROLLER ◄──► USER INTERFACE 350

# FIG. 17

CATHETER
INSERTING POSITION

170

CATHETER
MOVING PATH

BIFURCATION

CROSSOVER

LESION

ROI

# FIG. 18

```
           START

             │
             ▼
   ┌─────────────────────┐
   │    USER SETTING     │ ～S1810
   └─────────────────────┘
             │
             ▼
   ┌─────────────────────┐
   │   ANALYZE BLOOD     │ ～S1820
   │  VESSEL INFORMATION │
   └─────────────────────┘
             │
             ▼
   ┌─────────────────────┐
   │ CATHETER MOVING INFORMATION │ ～S1830
   └─────────────────────┘
             │
             ▼
   ┌─────────────────────┐
   │  PROVIDE CATHETER   │ ～S1840
   │  MOVING INFORMATION │
   └─────────────────────┘
             │
             ▼
            END
```

# FIG. 19

(A)

(B)

# FIG. 20

400

410 420 430

| IMAGE SIGNAL ACQUIRER | ←→ | CONTROLLER | ←→ | DISPLAY |

# FIG. 21

BIFURCATION: N
BLOOD VESSEL
PARTED ANGLE: ΘN

BIFURCATION: N1
BLOOD VESSEL
PARTED ANGLE: ΘN1

Dn_n1

Dn_n2

BIFURCATION: N2
BLOOD VESSEL
PARTED ANGLE: ΘN2

# FIG. 22

BIFURCATION N

220

ROI

220

BIFURCATION N1

E(Θ1)

E(Θ2)

E(Θ3)

........

E(Θi - 1)

E(Θi)

# FIG. 23

```
                    ┌─────────────┐
                    │    START    │
                    └─────────────┘
                           │
                           ▼
          ┌─────────────────────────────┐
          │   DETECT BLOOD VESSEL       │  ～S2310
          │   INFORMATION IN IMAGE 1    │
          └─────────────────────────────┘
                           │
                           ▼
          ┌─────────────────────────────┐
          │   DETECT BLOOD VESSEL       │  ～S2320
          │   INFORMATION IN IMAGE 2    │
          └─────────────────────────────┘
                           │
                           ▼
          ┌─────────────────────────────┐
          │   DETECT AND COMPARE        │  ～S2330
          │   FEATURE VALUES            │
          └─────────────────────────────┘
                           │
                           ▼
          ┌─────────────────────────────┐
          │   DESIGNATING LANDMARKS     │  ～S2340
          │   OF IMAGE REGISTRATION     │
          └─────────────────────────────┘
                           │
                           ▼
                    ┌─────────────┐
                    │     END     │
                    └─────────────┘
```